# EUROPÄISCHE PATENTSCHRIFT

(11) **EP 2 906 545 B1**
(45) Veröffentlichungstag und Bekanntmachung des Hinweises auf die Patenterteilung: **21.09.2016**
(21) Anmeldenummer: 13774693.9
(22) Anmeldetag: 14.10.2013
(51) Int. Cl.: C07D 309/04, C07D 309/10

(54) **VERFAHREN ZUR INTEGRIERTEN HERSTELLUNG VON 2-SUBSTITUIERTEN 4-HYDROXY-4-METHYL-TETRAHYDROPYRANEN UND VON 2-SUBSTITUIERTEN 4-METHYL-TETRAHYDROPYRANEN**
METHOD FOR INTEGRATED PRODUCTION OF 2-SUBSTITUTED 4-HYDROXY-4-METHYL TETRAHYDROPYRANS AND OF 2-SUBSTITUTED 4-METHYL TETRAHYDROPYRANS
PROCÉDÉ DE PRODUCTION INTÉGRÉE DE 4-HYDROXY-4-MÉTHYLTÉTRAHYDRO-PYRANES 2-SUBSTITUÉS ET DE 4-MÉTHYLTÉTRAHYDROPYRANES 2-SUBSTITUÉS

(30) Priorität: 15.10.2012 EP 12188518
(43) Veröffentlichungstag der Anmeldung: 19.08.2015
(73) Patentinhaber: BASF SE, 67056 Ludwigshafen am Rhein (DE)
(72) Erfinder: STORK, Timon, 68642 Bürstadt Bobstadt (DE); RÖDER, Oskar, 67377 Gommersheim (DE); EBEL, Klaus, 68542 Heddesheim (DE); PELZER, Ralf, 37699 Fürstenberg (DE); KRAUSE, Wolfgang, 68782 Brühl-Rohrhof (DE); BECK, Karl, 76684 Östringen (DE)
(86) Internationale Anmeldenummer: PCT/EP2013/071409
(87) Internationale Veröffentlichungsnummer: WO 2014/060345

(56) Entgegenhaltungen:
- EP-A1- 1 927 593
- WO-A1-2010/133473
- WO-A1-2011/147919
- WO-A1-2011/154330
- PANKAJ GUPTA ET AL: "Odoriferous cyclic ethers via co-halogenation reaction: facile preparation of nerol oxide, florol, florol methyl ether, and pytyol methyl ether", HELVETICA CHIMICA ACTA, VERLAG HELVETICA CHIMICA ACTA, Bd. 90, 1. Januar 2007 (2007-01-01), Seiten 196-204, XP002661260, ISSN: 0018-019X
- ALEXANDRA MACEDO ET AL: "Solvent-free catalyzed synthesis of tetrahydropyran odorants: the role of SiO2.p-TSA catalyst on the Prins-cyclization reaction", JOURNAL OF THE BRAZILIAN CHEMICAL SOCIETY, SAO PAULO, BR, Bd. 21, Nr. 8, 4. Mai 2010 (2010-05-04), Seiten 1563-1571, XP002661261, ISSN: 0103-5053, DOI: 10.1590/S0103-50532010000800023
- GEVORKN A A ET AL: "Mechanism of cycloalkylation of allylcarbinols with aldehydes and ketones", CHEMISTRY OF HETEROCYCLIC COMPOUNDS, SPRINGER NEW YORK LLC, US, Nr. 12, 1. Januar 1982 (1982-01-01), Seiten 1240-1242, XP002597060, ISSN: 0009-3122

## Beschreibung

### HINTERGRUND DER ERFINDUNG

Die vorliegende Erfindung betrifft ein Verfahren zur integrierten Herstellung von 2-substituierten 4-Hydroxy-4-methyl-tetrahydropyranen und von 2-substituierten 4-Methyl-tetrahydropyranen.

### STAND DER TECHNIK

2-substituierte 4-Hydroxy-4-methyl-tetrahydropyrane sind wertvolle Verbindungen für den Einsatz als Aromachemikalien. So zeichnet sich beispielsweise das cis/trans-Diastereomerengemisch des 2-(2-Methylpropyl)-4-hydroxy-4-methyl-tetrahydropyrans 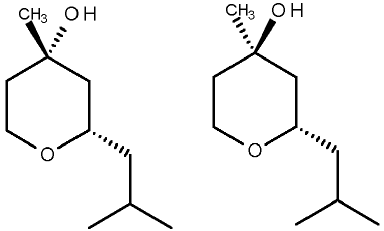 durch einen angenehmen Maiglöckchenduft aus und ist in besonderem Maße zur Verwendung als Aromachemikalie, z. B. zur Herstellung von Riechstoffkompositionen, geeignet.

Die EP 1 493 737 A1 offenbart ein Verfahren zur Herstellung von Gemischen von ethylenisch ungesättigten 4-Methyl- bzw. 4-Methylenpyranen und den entsprechenden 4-Hydroxypyranen durch Umsetzung der entsprechenden Aldehyde mit Isoprenol, wobei die Umsetzung in einem Reaktionssystem initiiert wird, in dem das molare Verhältnis von Aldehyd zu Isoprenol größer als 1 ist, d. h. der Aldehyd im Überschuss eingesetzt wird. Darüber hinaus offenbart das Dokument die anschließende Dehydratisierung der genannten Gemische zu den gewünschten ethylenisch ungesättigten Pyranen. Als geeignete Katalysatoren für den ersten Reaktionsschritt werden Mineralsäuren wie Salzsäure oder Schwefelsäure, bevorzugt jedoch Methansulfonsäure oder p-Toluolsulfonsäure genannt.

EP 1 516 879 A1 offenbart ein Verfahren zur Herstellung von ethylenisch ungesättigten 4-Methyl- bzw. 4-Methylenpyranen durch Umsetzung eines entsprechenden Aldehyds mit Isoprenol unter dehydratisierenden Bedingungen, wobei die Wassermenge im Reaktor bis zu 0,25 Gew.-% beträgt, während der Umsatz der im Unterschuss eingesetzten Ausgangsverbindung weniger als 50 % beträgt. Als hierfür geeignete Katalysatoren werden ebenfalls Mineralsäuren wie Salzsäure oder Schwefelsäure, bevorzugt jedoch Methansulfonsäure oder p-Toluolsulfonsäure genannt.

Die WO 2010/133473 beschreibt ein Verfahren zur Herstellung von 2-substituierten 4-Hydroxy-4-methyl-tetrahydropyranen der Formel (I) 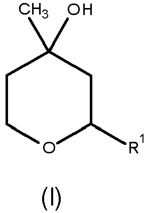 wobei der Rest R¹ für einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen steht, bei dem man Isoprenol (3-Methylbut-3-en-1-ol) mit einem Aldehyd der Formel R¹-CHO umsetzt, wobei man die Umsetzung in Gegenwart von Wasser und in Gegenwart eines stark sauren Kationenaustauschers durchführt.

Die WO 2011/154330 beschreibt ein zur WO 2010/133473 vergleichbares Verfahren, wobei das erhaltene Reaktionsgemisch einer destillativen Aufarbeitung in einer Trennwandkolonne oder in zwei thermisch gekoppelten Destillationskolonnen durchgeführt wird.

Wie in der WO 2010/133473 und WO 2011/154330 beschrieben ist, fällt bei der sauer katalysierten Umsetzung von Isoprenol (3-Methylbut-3-en-1-ol) mit einem Aldehyd der Formel R¹-CHO ein komplexes Reaktionsgemisch an, das neben 2-substituierten 4-Hydroxy-4-methyltetrahydropyranen noch dehydratisierte Nebenprodukte der Formeln (A), (B) und/oder (C) 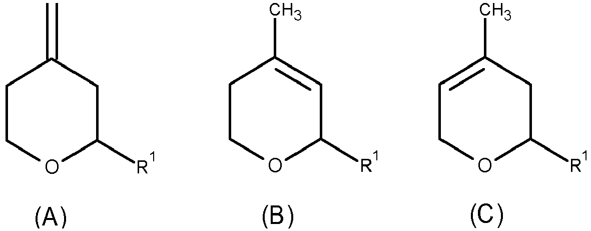 sowie als weitere Nebenprodukte Acetale (D) und 1,3-Dioxane (E) 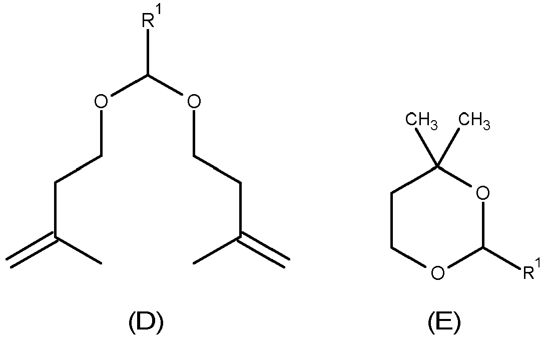 enthält. Diese Nebenprodukte können bislang nicht zur Bereitstellung weiterer Wertstoffe genutzt werden, sondern werden entweder ausgeschleust oder gemeinsam mit den im Überschuss eingesetzten Ausgangsverbindungen wieder in die Umsetzung von Isoprenol mit dem Aldehyd zurückgeführt. Letzteres ist aufgrund einer möglichen Aufpegelung dieser Komponenten im Reaktionsgemisch nicht unproblematisch.

Die WO 2011/147919 beschreibt ein Verfahren zur Herstellung von 2-substituierten 4-Hydroxy-4-methyl-tetrahydropyranolen und speziell von 2-Isobutyl-4-hydroxy-4-methyl-tetrahydropyran durch Umsetzung von Isoprenol mit Prenal und anschließender Hydrierung.

Eine weitere wertvolle Aromachemikalie ist 2-(2-Methylpropyl)-4-methyl-tetrahydro-2H-pyran, das auch als Dihydrorosenoxid bezeichnet wird.

Dihydrorosenoxid wurde das erste Mal aus bulgarischem Rosenöl isoliert und daraufhin durch Julia und Jacquet synthetisch dargestellt (Julia, M.; Jacquet, B., Bulletin de la Societe Chimique de France 1963, 8-9, 1983). Ausgehend von But-2-en-1-al wurde durch eine Diels-Alder-Reaktion mit Ethyl-Vinyl-Ether und anschließender Hydrierung ein cyclisches Acetal erhalten. Nach Abspaltung von Ethanol, Hydrobromierung der erhaltenen Doppelbindung und abschließender Grignard-Reaktion mit Isopropylmagnesiumbromid konnte die Mischung aus cis- und trans-Dihydrorosenoxid das erste Mal synthetisch dargestellt werden.
J. H. P. Tyman und B. J. Willis beschreiben in Tetrahedron Letters Nr. 51, 4507 - 4508, 1970, die säurekatalysierte Umsetzung von 3-Alken-1-olen mit Aldehyden, speziell die Umsetzung von 3-Methyl-2-buten-1-al mit 2-Methyl-1-buten-4-ol und anschließende Dehydratisierung. Das so erhaltene, eine exocyclische Methylengruppe aufweisende Zwischenprodukt wurde homogenkatalytisch in Gegenwart von SnCl₂/H₂PtCl₆ zum racemischen cis-2-(2-Methyl-prop-1-en-yl)-4-methyl-tetrahydropyran hydriert.

A. Machado et. al. beschreiben in Journal of the Brazilian Chemical Society Vol. 21 Nr. 8, 1563-1571, 2010 eine weiteres Verfahren zur säurekatalysierten Umsetzung von 2-Methyl-1-buten-4-ol mit unteschiedlichen Aldehyden.

Der vorliegenden Erfindung liegt die Aufgabe zugrunde, ein verbessertes Verfahren zur Herstellung 2-substituierter 4-Hydroxy-4-methyl-tetrahydropyrane zur Verfügung zu stellen, das auch eine möglichst effektive Nutzung der bisher nicht verwertbaren Nebenprodukte ermöglicht.
Überraschenderweise wurde jetzt gefunden, dass sich der bei der sauer katalysierten Herstellung von 2-substituierten 4-Hydroxy-4-methyl-tetrahydropyranen durch Umsetzung von Isoprenol (3-Methylbut-3-en-1-ol) mit einem geeigneten Aldehyd anfallende Nebenprodukt-haltige Seitenstrom (= Abfallstrom) zur Herstellung von 2-substituierten 4-Methyl-tetrahydropyranen und speziell zur Herstellung von Dihydrorosenoxid eignet. Dabei ist insbesondere problematisch, dass die Hauptkomponenten des Seitenstroms, die drei isomeren Dihydropyranole (A), (B) und (C) einerseits und das Dioxan (E) andererseits nicht mit vertretbarem Aufwand destillativ getrennt werden können. Überraschenderweise wurde jedoch gefunden, dass durch Hydrierung des gesamten Seitenstroms die isomeren Dihydropyranole (A), (B) und (C) in 2-substituierte 4-Methyl-tetrahydropyrane überführt werden können, welche sich dann von dem nicht verwertbaren Dioxan (E) destillativ abtrennen lassen. Somit wird ein integriertes Verfahren zur gleichzeitigen Herstellung von 2-substituierten 4-Hydroxy-4-methyl-tetrahydropyranen und von 2-substituierten 4-Methyl-tetrahydropyranen bereitgestellt. Somit kann ein Großteil des bisherigen Seitenstroms auch der Verwendung als Aromachemikalie und speziell als Duftstoff zugeführt werden.

### ZUSAMMENFASSUNG DER ERFINDUNG

Ein erster Gegenstand der Erfindung ist ein Verfahren zur Herstellung von 2-substituierten 4-Hydroxy-4-methyl-tetrahydropyranen der allgemeinen Formel (I) und von 2-substituierten 4-Methyl-tetrahydropyranen der allgemeinen Formel (II) 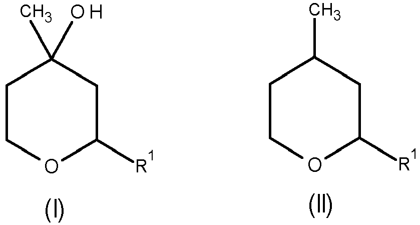 worin
- R¹: für geradkettiges oder verzweigtes C₁-C₁₂-Alkyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) 3-Methylbut-3-en-1-ol der Formel (III) 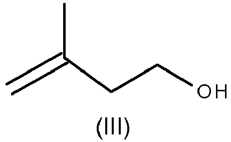 mit einem Aldehyd der Formel (IV)

   R¹-CHO (IV)

   wobei R¹ in der Formel (IV) die zuvor angegebene Bedeutung hat, in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (I), wenigstens eine der Verbindungen (V.1), (V.2) oder (V.3) und wenigstens eine Dioxanverbindung (VI) enthält 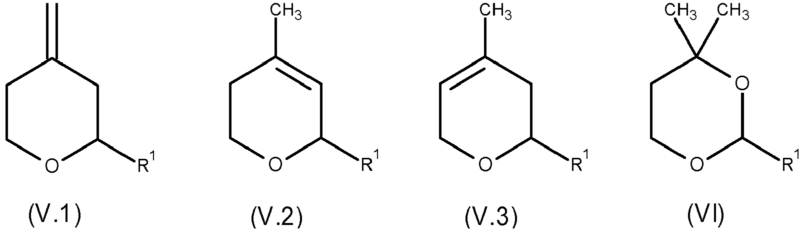 wobei R¹ in der Formel (VI) die zuvor angegebene Bedeutung hat,
b) das Reaktionsprodukt aus Schritt a) einer Auftrennung unter Erhalt einer an 2-substituierten 4-Hydroxy-4-methyl-tetrahydropyranen der allgemeinen Formel (I) angereicherten Fraktion und einer Fraktion, die wenigstens eine der Verbindungen (V.1), (V.2) oder (V.3) und wenigstens eine Dioxanverbindung (VI) enthält, unterzieht,
c) die Fraktion, die wenigstens eine der Verbindungen V.1), (V.2) oder (V.3) und wenigstens eine Dioxanverbindung (VI) enthält, einer Hydrierung unterzieht,
d) aus dem in Schritt c) erhaltenen Hydrierprodukt eine an 2-substituierten 4-Methyl-tetrahydropyranen (II) angereicherte Fraktion und eine an der wenigstens einen Dioxanverbindung (VI) angereicherte Fraktion isoliert.

### BESCHREIBUNG DER ERFINDUNG

Das erfindungsgemäße Verfahren weist die folgenden Vorteile auf:
- Ein Großteil des bisherigen Seitenstroms (Abfallstroms) bei der sauer katalysierten Herstellung von 2-substituierten 4-Hydroxy-4-methyl-tetrahydropyranen kann als Wertstoff verwendet werden.
- Mit der erfindungsgemäß vorgesehenen Hydrierung wird ein Zugang zu 2-substituierten 4-Methyl-tetrahydropyranen und speziell zu Dihydrorosenoxid ermöglicht, der nur eine Reaktionsstufe, ausgehend vom Seitenstrom, erfordert.
- Die im Reaktionsgemisch der Herstellung von 2-substituierten 4-Hydroxy-4-methyl-tetrahydropyranen enthaltenen nicht verwertbaren Dioxane können nach der Hydrierung wirkungsvoll vom Wertprodukt abgetrennt werden.
- Zur Herstellung der 2-substituierten 4-Methyl-tetrahydropyrane, speziell des Dihydrorosenoxids, müssen keine weiteren teuren und/oder potentiell gefährlichen Reagenzien, wie etwa Grignard-Reagenzien oder komplexe Hydride, wie Lithiumaluminiumhydrid, eingesetzt werden.

Sofern im Folgenden nicht genauer angegeben, bezeichnen die Begriffe "2-substituiertes 4-Hydroxy-4-methyl-tetrahydropyran",
"2-substituiertes 4-Methyl-tetrahydropyran",
"2-(2-Methylpropyl)-4-hydroxy-4-methyl-tetrahydropyran",
"2-(2-Methylpropyl)-4-methyl-tetrahydropyran" (= "Dihydrorosenoxid"),
im Rahmen der Erfindung cis/trans-Gemische jedweder Zusammensetzung sowie die reinen Konformations-Isomere. Die zuvor genannten Begriffe bezeichnen weiterhin alle Enantiomere in Reinform sowie racemische und optisch aktive Gemische der Enantiomeren dieser Verbindungen.

Sofern im Folgenden von cis- und trans-Diastereomeren der Verbindungen (I) oder (II) die Rede ist, wird jeweils nur eine der enantiomeren Formen abgebildet. Lediglich zur Veranschaulichung werden im Folgenden die Isomeren des 2-(2-Methylpropyl)-4-methyl-tetrahydropyrans (II) (Dihydrorosenoxid) wiedergegeben:

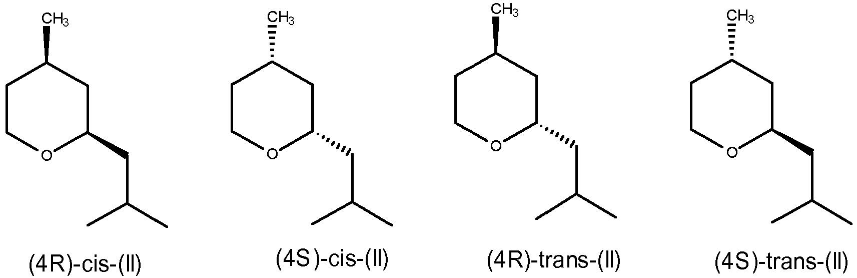

Im Rahmen der vorliegenden Erfindung steht der Ausdruck geradkettiges oder verzweigtes Alkyl vorzugsweise für C₁-C₆-Alkyl und besonders bevorzugt für C₁-C₄-Alkyl. Alkyl steht insbesondere für Methyl, Ethyl, n-Propyl, Isopropyl, n-Butyl, Isobutyl (2-Methylpropyl), sec.-Butyl (1-Methylpropyl), tert.-Butyl (1,1-Dimethylethyl), n-Pentyl oder n-Hexyl. Speziell steht Alkyl für Methyl, Ethyl, n-Propyl, Isopropyl, oder Isobutyl.

Im Rahmen der vorliegenden Erfindung steht der Ausdruck geradkettiges oder verzweigtes Alkoxy vorzugsweise für C₁-C₆-Alkoxy und besonders bevorzugt für C₁-C₄-Alkoxy. Alkoxy steht insbesondere für Methoxy, Ethoxy, n-Propyloxy, Isopropyloxy, n-Butyloxy, Isobutyloxy, sec.-Butyloxy, tert.-Butyloxy, n-Pentyloxy oder n-Hexyloxy. Speziell steht Alkoxy für Methoxy, Ethoxy, n-Propyloxy, Isopropyloxy, oder Isobutyloxy. Im Rahmen der Erfindung bezeichnet Cycloalkyl einen cycloaliphatischen Rest mit vorzugsweise 3 bis 10, besonders bevorzugt 5 bis 8, Kohlenstoffatomen. Beispiele für Cycloalkylgruppen sind insbesondere Cyclopropyl, Cyclobutyl, Cyclopentyl, Cyclohexyl, Cycloheptyl oder Cyclooctyl. Speziell steht Cycloalkyl für Cyclohexyl.

Substituierte Cycloalkylgruppen können in Abhängigkeit von der Ringgröße einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Die Cycloalkylgruppen tragen im Falle einer Substitution vorzugsweise eine oder mehrere, beispielsweise eine, zwei, drei, vier oder fünf C₁-C₆-Alkylgruppen. Beispiele für substituierte Cycloalkylgruppen sind insbesondere 2- und 3-Methylcyclopentyl, 2- und 3-Ethylcyclopentyl, 2-, 3- und 4-Methylcyclohexyl, 2-, 3- und 4-Ethylcyclohexyl, 2-, 3-und 4-Propylcyclohexyl, 2-, 3- und 4-Isopropylcyclohexyl, 2-, 3- und 4-Butylcyclohexyl und 2-, 3- und 4-Isobutylcyclohexyl.

Der Ausdruck "Aryl" umfasst im Rahmen der vorliegenden Erfindung ein- oder mehrkernige aromatische Kohlenwasserstoffreste mit üblicherweise 6 bis 18, vorzugsweise 6 bis 14, besonders bevorzugt 6 bis 10 Kohlenstoffatomen. Beispiele für Aryl sind insbesondere Phenyl, Naphthyl, Indenyl, Fluorenyl, Anthracenyl, Phenanthrenyl, Naphthacenyl, Chrysenyl, Pyrenyl, etc., und speziell Phenyl oder Naphthyl.

Substituierte Aryle können in Abhängigkeit von der Anzahl und Größe ihrer Ringsysteme einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Beispiele für substituierte Arylreste sind 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl.

### Schritt a)

Einer der Ausgangsstoffe für Schritt a) des erfindungsgemäßen Verfahrens ist 3-Methylbut-3-en-1-ol (Isoprenol) der Formel (III), 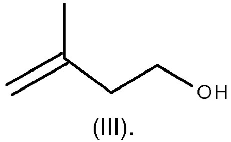 Isoprenol ist nach bekannten Verfahren aus Isobuten und Formaldehyd in jedem Maßstab gut zugänglich und kommerziell verfügbar. An die Reinheit, Qualität oder Herstellverfahren des erfindungsgemäß einzusetzenden Isoprenols sind keine besonderen Anforderungen zu stellen. Es kann in handelsüblicher Qualität und Reinheit in Schritt a) des erfindungsgemäßen Verfahrens eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew.-% oder noch mehr bevorzugt 98 bis 99,8 Gew.-%.

Ein weiterer Ausgangsstoff für Schritt a) des erfindungsgemäßen Verfahrens ist ein Aldehyd der Formel (IV) R¹-CHO, wobei R¹ in der Formel (IV) die zuvor angegebene Bedeutung hat.

Bevorzugt steht R¹ in den Verbindungen der Formel (I), (II), (IV), (V.1), (V.2), (V.3) und (VI) für geradkettiges oder verzweigtes C₁-C₁₂-Alkyl.

Besonders bevorzugt steht R¹ für geradkettiges oder verzweigtes C₁-C₆-Alkyl. In einer weiteren bevorzugten Ausführung steht R¹ für Phenyl.

Erfindungsgemäß bevorzugte Bedeutungen für den Rest R¹ sind somit beispielsweise Methyl, Ethyl, n-Propyl, Isopropyl, n-Butyl, Isobutyl, n-Pentyl, n-Hexyl oder n-Heptyl, bevorzugt Methyl, Ethyl, n-Propyl, Isopropyl, n-Butyl, Isobutyl, ganz besonders bevorzugt Isobutyl (2-Methylpropyl).

Bevorzugt einzusetzende Aldehyde der Formel (IV) sind: Acetaldehyd, Valeraldehyd, Isovaleraldehyd, Pentanal, Hexanal, Heptanal, Benzaldehyd, Citral, Citronellal. Erfindungsgemäß ganz besonders bevorzugt einzusetzende Aldehyde der Formel (IV) sind Isovaleraldehyd und Benzaldehyd, insbesondere Isovaleraldehyd.

Die vorliegende Erfindung betrifft somit im Rahmen einer bevorzugten Ausführungsform ein Verfahren zur Herstellung und Isolierung von 2-(2-Methylpropyl)-4-hydroxy-4-methyl-tetrahydropyran der Formel (Ia) und von 2-(2-Methylpropyl)-4-methyl-tetrahydropyran der Formel (IIa) (Dihydrorosenoxid)

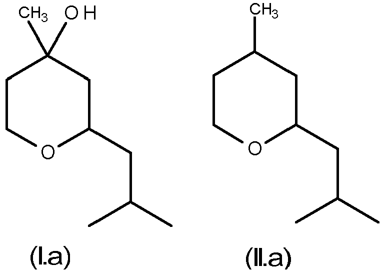

Bevorzugt werden in Schritt a) das 3-Methylbut-3-en-ol (III) und der Aldehyd (IV) in einem molaren Verhältnis von etwa 1 zu 2 bis 2 zu 1, besonders bevorzugt von 0,7 zu 1 bis 2 zu 1, insbesondere von 1 zu 1 bis 2 zu 1, eingesetzt. In einer speziellen Ausführung werden in Schritt a) das 3-Methylbut-3-en-ol (III) und der Aldehyd (IV) in einem molaren Verhältnis von 1 zu 1 bis 1,5 zu 1 eingesetzt.

Erfindungsgemäß erfolgt die Umsetzung in Schritt a) in Gegenwart eines sauren Katalysators. Prinzipiell kann für die Umsetzung in Schritt a) jeder saure Katalysator verwendet werden, d. h., jede Substanz, die Brönstedt- oder Lewis-Acidität aufweist. Beispiele für geeignete Katalysatoren sind Protonensäuren, wie Salzsäure, Schwefelsäure, Phosphorsäure, Methansulfonsäure und p-Toluolsulfonsäure, saure molekulare Elementverbindungen, wie Aluminiumchlorid, Bortrifluorid, Zinkchlorid, Phosphorpentafluorid, Arsentrifluorid, Zinntetrachlorid, Titantetrachlorid und Antimonpentafluorid; oxidische saure Festkörper wie Zeolithe, Silikate, Aluminate, Alumosilikate, Tone und saure lonentauscher.

Bevorzugt ist der in Schritt a) eingesetzte saure Katalysator ausgewählt unter Salzsäure, Schwefelsäure, Methansulfonsäure, p-Toluolsulfonsäure und stark sauren Kationenaustauschern.

In einer ersten Variante erfolgt die Umsetzung in Schritt a) in Gegenwart einer Brönstedt-Säure, die vorzugsweise ausgewählt ist unter Salzsäure, Schwefelsäure, Methansulfonsäure, p-Toluolsulfonsäure. Bevorzugt wird in dieser ersten Variante in Schritt a) ein Lösungsmittel eingesetzt, das vorzugsweise ausgewählt ist unter Kohlenwasserstoffen und Kohlenwasserstoffgemischen. Geeignete Lösungsmittel sind beispielsweise Hexan, Heptan, Ligroin, Petrolether, Cyclohexan, Dekalin, Toluol, Xylol und Mischungen davon. In dieser ersten Variante beträgt der Wassergehalt des Reaktionsgemisches höchstens 0,25 Gew.-%, besonders bevorzugt höchstens 2 Gew.-%, bezogen auf das Gesamtgewicht des Reaktionsgemisches. Im Verlauf der Umsetzung in Schritt a) kann Wasser gebildet werden, z. B. durch die Dehydratisierung des Verfahrensproduktes der Formel (I) als mögliche Nebenreaktion. Um dennoch den Wassergehalt gering zu halten, kann das gebildete Wasser gemeinsam mit dem eingesetzten Lösungsmittel abdestilliert, das Wasser vom Lösungsmittel nach üblichen Verfahren zumindest teilweise abgetrennt und anschließend das Lösungsmittel in den Reaktionsschritt a) zurückgeführt werden. Bevorzugt wird der Katalysator in dieser ersten Variante in einer Menge von 0,05 bis 5 Mol-%, besonders bevorzugt von 0,1 bis 4 Mol-%, bezogen auf den Aldehyd (IV) eingesetzt. Bevorzugt erfolgt die Umsetzung in Schritt a) nach dieser ersten Variante bei einer Temperatur im Bereich von 20 bis 120 °C, besonders bevorzugt 40 bis 110 °C.

In einer zweiten Variante erfolgt die Umsetzung in Schritt a) in Gegenwart eines stark sauren Kationenaustauschers. Unter dem Begriff stark saurer Kationenaustauscher wird dabei ein Kationenaustauscher in der H⁺-Form verstanden, der stark saure Gruppen aufweist. Bei den stark sauren Gruppen handelt es sich in der Regel um Sulfonsäuregruppen. Die sauren Gruppen sind in der Regel angebunden an eine Polymermatrix, die z. B. gelförmig bzw. makroporös sein kann. Eine bevorzugte Ausführungsform des erfindungsgemäßen Verfahrens ist dementsprechend dadurch gekennzeichnet, dass man einen stark sauren, Sulfonsäuregruppen aufweisenden Kationenaustauscher einsetzt. Geeignete stark saure Kationenaustauscher sind in der WO 2010/133473 und WO 2011/154330 beschrieben, worauf hier in vollem Umfang Bezug genommen wird.

Geeignet für den Einsatz in Schritt a) sind stark saure Ionenaustauscher (wie z. B. Amberlyst, Amberlite, Dowex, Lewatit, Purolite, Serdolit), die auf Polystyrol basieren und die Copolymere aus Styrol und Divinylbenzol als Trägermatrix mit Sulfonsäuregruppen in H⁺-Form enthalten sowie mit Sulfonsäuregruppen (-SO₃H) funktionalisierte Ionenaustauschergruppen. Die Ionenaustauscher unterscheiden sich im Aufbau ihrer Polymergerüste, und man unterscheidet gelförmige und makroporöse Harze. In einer speziellen Ausführung wird in Schritt a) ein perfluoriertes polymeres Ionenaustauscherharz eingesetzt. Derartige Harze werden z. B. unter der Bezeichnung Nafion ® von der Firma DuPont vertrieben. Als Beispiel für ein solches perfluoriertes polymeres Ionenaustauscherharz sein Nafion ® NR-50 genannt.

Für die Umsetzung in Schritt a) geeignete kommerziell verfügbare stark saure Kationenaustauscher sind beispielsweise unter den Handelsnamen Lewatit ® (Lanxess), Purolite ® (The Purolite Company), Dowex ® (Dow Chemical Company), Amberlite ® (Rohm and Haas Company), Amberlyst (TM) (Rohm and Haas Company) bekannt. Bevorzugte stark saure Kationenaustauscher sind: Lewatit ® K 1221, Lewatit ® K 1461, Lewatit ® K 2431, Lewatit ® K 2620, Lewatit ® K 2621, Lewatit ® K 2629, Lewatit ® K 2649, Amberlite ® FPC 22, Amberlite ® FPC 23, Amberlite ® IR 120, Amberlyst (TM) 131, Amberlyst (TM) 15, Amberlyst (TM) 31, Amberlyst (TM) 35, Amberlyst (TM) 36, Amberlyst (TM) 39, Amberlyst (TM) 46, Amberlyst (TM) 70, Purolite ® SGC650, Purolite ® C100H, Purolite ® C150H, Dowex ® 50X8, Serdolit ® rot und Nation ® NR-50.

Die stark sauren lonentauscherharze werden in der Regel mit Salzsäure und/oder Schwefelsäure regeneriert.

In einer speziellen Ausführung werden in Schritt a) das 3-Methylbut-3-en-ol (III) und der Aldehyd (IV) in Gegenwart eines stark sauren Kationenaustauschers und in Gegenwart von Wasser umgesetzt. Prinzipiell kann das Reaktionsgemisch in Schritt a) bereits geringe Mengen Wasser enthalten, das durch die Dehydratisierung des Verfahrensproduktes der Formel (I) als mögliche Nebenreaktion freigesetzt werden kann. Nach einer speziellen Ausführung wird dem Reaktionsgemisch neben Isoprenol (III) und dem Aldehyd der Formel (IV) sowie etwaigem Wasser aus der Reaktion zusätzlich noch Wasser zugesetzt.

Üblicherweise führt man die Umsetzung des Isoprenols (III) mit dem Aldehyd der Formel (IV) in Gegenwart von etwa mindestens 10 mol-% zugesetztem Wasser durch, wobei sich die Menge an Wasser auf die Menge des jeweils im Unterschuss eingesetzten Ausgangsstoffes Isoprenol (III) oder Aldehyd (IV), oder, in dem Fall der äquimolaren Umsetzung der beiden Ausgangsstoffe (III) und (IV), auf die Stoffmenge eines der beiden bezieht.

Oberhalb des angegebenen Wertes kann die Menge an Wasser frei gewählt werden und ist, wenn überhaupt, nur durch verfahrenstechnische oder ökonomische Aspekte begrenzt und kann durchaus in großem, beispielsweise in 10- bis 100-fachem Überschuss oder auch darüber eingesetzt werden. Vorzugsweise bereitet man ein Gemisch aus Isoprenol (III) und dem Aldehyd der Formel (IV), vorzugsweise Isovaleraldehyd, mit der zuzusetzenden Menge Wasser, so dass das zugegebene Wasser in dem Gemisch aus Isoprenol und dem Aldehyd gelöst bleibt d. h. kein zweiphasiges System vorliegt.

Üblicherweise setzt man im Rahmen dieser Ausführungsform des erfindungsgemäßen Verfahrens die Ausgangsstoffe Isoprenol (III) und den Aldehyd der Formel (IV) in Gegenwart von mindestens 25 mol-%, bevorzugt von mindestens 50 mol-%, noch mehr bevorzugt von mindestens 75 und noch mehr bevorzugt von mindestens 90 mol-% zugesetztem Wasser um, wobei sich die Menge an Wasser auf die Menge des jeweils im Unterschuss eingesetzten Ausgangsstoffes Isoprenol (III) oder Aldehyd (IV), oder, in dem Fall der äquimolaren Umsetzung der beiden Ausgangsstoffe (III) und (IV), auf die Stoffmenge eines der beiden bezieht.

Vorzugsweise setzt man im Rahmen dieser Ausführungsform des erfindungsgemäßen Verfahrens die Ausgangsstoffe Isoprenol (III) und den Aldehyd der Formel (IV) in Gegenwart von bis etwa 1000 mol-% Wasser um, wobei sich die Menge an Wasser auf die Menge des jeweils im Unterschuss eingesetzten Ausgangsstoffes Isoprenol (III) oder Aldehyd (IV), oder, in dem Fall der äquimolaren Umsetzung der beiden Ausgangsstoffe (III) und (IV), auf die Stoffmenge eines der beiden bezieht.

Im Rahmen einer bevorzugten Ausführungsform führt man die erfindungsgemäß durchzuführende Umsetzung so durch, dass man sie in Gegenwart einer mindestens äquimolaren Menge an zugesetztem Wasser durchführt, wobei sich die Menge an Wasser auf die Menge des jeweils im Unterschuss eingesetzten Ausgangsstoffes Isoprenol (III) oder Aldehyd (IV), oder, in dem Fall der äquimolaren Umsetzung der beiden Ausgangsstoffe (III) und (IV), auf die Stoffmenge eines der beiden bezieht. Demzufolge führt man die erfindungsgemäße Umsetzung von Isoprenol mit dem gewählten Aldehyd der Formel (IV) bevorzugt in Gegenwart von 90 bis 250 mol-%, besonders bevorzugt 90 bis 230 mol-%, noch mehr bevorzugt 90 bis 200 mol-% und am meisten bevorzugt in Gegenwart von 90 bis 180 mol-% Wasser durch, wobei sich die Menge an Wasser auf die Menge des jeweils im Unterschuss eingesetzten Ausgangsstoffes Isoprenol (III) oder Aldehyd (IV), oder, in dem Fall der äquimolaren Umsetzung der beiden Ausgangsstoffe (III) und (IV), auf die Stoffmenge eines der beiden bezieht.

Zur Umsetzung von Isoprenol (III) mit dem Aldehyd (IV) in Schritt a) kann man die genannten Ausgangsstoffe und gegebenenfalls das zugesetzte Wasser mit dem sauren Kationenaustauscher in Kontakt bringen. Vorzugsweise werden Isoprenol (III), Aldehyd (IV) und gegebenenfalls das zugesetzte Wasser in Form eines Gemisches in Schritt a) eingesetzt. Die genannten Ausgangsstoffe, d. h. Isoprenol (III) und der Aldehyd (IV) und das in der vorstehenden Menge einzusetzende Wasser können in beliebiger Reihenfolge miteinander in Kontakt gebracht bzw. gemischt werden.

Die Menge an stark saurem Kationenaustauscher in Schritt a) ist nicht kritisch und kann unter Berücksichtigung des wirtschaftlichen und verfahrenstechnischen Aspektes in breiten Grenzen frei gewählt werden. Die Umsetzung kann dementsprechend sowohl in Gegenwart katalytischer Mengen als auch in Gegenwart großer Überschüsse des stark sauren Kationenaustauschers durchgeführt werden. Üblicherweise setzt man den stark sauren Kationentauscher in einer Menge von etwa 5 bis etwa 40 Gew.-%, bevorzugt in einer Menge von etwa 20 bis etwa 40 Gew.- % und besonders bevorzugt in einer Menge von etwa 20 bis etwa 30 Gew.-%, jeweils bezogen auf die Summe an eingesetztem Isoprenol (III) und Aldehyd der Formel (IV), ein. Dabei beziehen sich die Angaben auf den gebrauchsfertigen Kationenaustauscher, der in der Regel mit Wasser vorbehandelt wird und dementsprechend Mengen von bis zu etwa 70 Gew.-%, bevorzugt von etwa 30 bis etwa 65 Gew.-% und besonders bevorzugt von etwa 40 bis etwa 65 Gew.-% Wasser umfassen kann. Insbesondere bei diskontinuierlicher Verfahrensführung kann sich daher ein darüber hinausgehender Wasserzusatz bei der Durchführung des erfindungsgemäßen Verfahrens erübrigen. Die genannten stark sauren Kationenaustauscher können in Schritt a) sowohl einzeln oder auch in Form von Gemischen eingesetzt werden.

Die Umsetzung in Schritt a) in Gegenwart eines stark sauren Kationenaustauschers kann wahlweise auch zusätzlich in Gegenwart eines unter den Reaktionsbedingungen inerten Lösungsmittels durchgeführt werden. Geeignete Lösungsmittel sind beispielsweise tert-Butylmethylether, Cyclohexan, Dekalin, Hexan, Heptan, Ligroin, Petrolether, Toluol oder Xylol. Die genannten Lösungsmittel können alleine oder in Form von Gemischen untereinander eingesetzt werden. Bevorzugt führt man die Umsetzung in Schritt a) in Gegenwart eines stark sauren Kationenaustauschers ohne Zusatz eines organischen Lösungsmittels durch.

Bevorzugt wird die Umsetzung von Isoprenol (III) mit dem gewählten Aldehyd (IV) in Schritt a) in Gegenwart von Wasser und in Gegenwart eines stark sauren Kationentauschers bei einer Temperatur im Bereich von 0 bis 70 °C, besonders bevorzugt bei einer Temperatur im Bereich von 10 bis 60 °C und insbesondere bei einer Temperatur im Bereich von 20 bis 50 °C durchgeführt. Dabei handelt es sich um die Temperatur des Reaktionsgemisches.

Die Umsetzung in Schritt a) kann diskontinuierlich oder kontinuierlich durchgeführt werden. Dabei kann man beispielsweise im diskontinuierlichen Fall die Umsetzung so vornehmen, dass man ein Gemisch von Isoprenol (III), dem Aldehyd (IV), gegebenenfalls Wasser und gegebenenfalls einem organischen Lösungsmittel in einem geeigneten Reaktionsgefäß vorlegt und den sauren Katalysator zugibt. Nach Abschluss der Reaktion kann dann der Katalysator durch geeignete Trennverfahren vom erhaltenen Reaktionsgemisch abgetrennt werden. Wird in Schritt a) eine Brönstedt-Säure, die vorzugsweise ausgewählt ist unter Salzsäure, Schwefelsäure, Methansulfonsäure, p-Toluolsulfonsäure, als Katalysator eingesetzt, so kann die Abtrennung des Katalysators z. B. nach wässriger Aufarbeitung destillativ erfolgen oder direkt destillativ erfolgen. Wird in Schritt a) ein stark saurer Kationenaustauscher als Katalysator eingesetzt, so kann die Abtrennung des Katalysators z. B. durch Filtration oder durch Zentrifugation erfolgen.

Im Rahmen einer bevorzugten Ausführungsform führt man die Umsetzung von Isoprenol (III) mit dem Aldehyd (IV) in Schritt a) kontinuierlich durch. Dazu kann beispielsweise eine Mischung der umzusetzenden Ausgangsstoffe Isoprenol und Aldehyd der Formel (III) mit Wasser bereitet werden und diese Mischung kontinuierlich mit einem stark sauren Kationentauscher in Kontakt gebracht werden. Dazu kann der gewählte Kationenaustauscher beispielsweise in einen geeigneten Durchflussreaktor, beispielsweise einen Rührreaktor mit Zu- und Ablauf oder einen Rohrreaktor eingebracht werden und die Ausgangsstoffe und das Wasser in diesen kontinuierlich ausgetragen werden und das Reaktionsgemisch kontinuierlich ausgetragen werden. Dabei können die Ausgangsstoffe und das Wasser wahlweise als Einzelkomponenten oder auch in Form eines wie vorstehend beschriebenen Gemisches in den Durchflussreaktor eingetragen werden.

Das in Schritt a) des erfindungsgemäßen Verfahrens erhaltene Reaktionsgemisch enthält neben den 2-substituierten 4-Hydroxy-4-methyl-tetrahydropyranen der Formel (I), 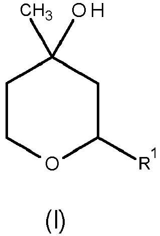 wenigstens eine der Verbindungen (V.1), (V.2) oder (V.3) und wenigstens eine Dioxanverbindung (VI) 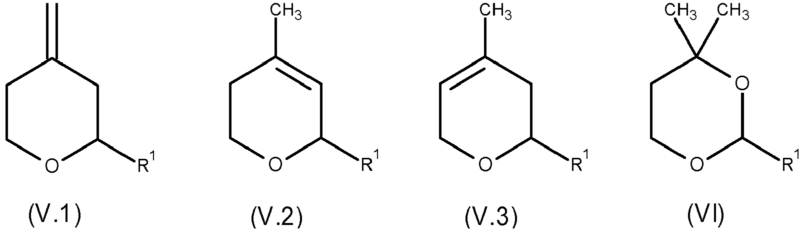 wobei R¹ in den Formeln (I), (V.1), (V.2), (V.3) und (VI) die zuvor angegebene Bedeutung hat. Bevorzugt steht R¹ für Isobutyl. In der Regel enthält das Reaktionsgemisch ein Gemisch der Verbindungen (V.1), (V.2) und (V.3).

Das in Schritt a) des erfindungsgemäßen Verfahrens erhaltene Reaktionsgemisch kann wenigstens ein weiteres Nebenprodukt erhalten, z. B. ein Acetal (VII) 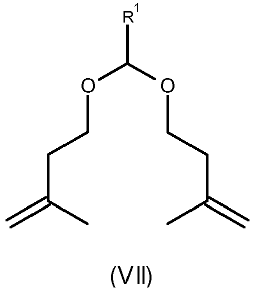 wobei R¹ die zuvor angegebene Bedeutung hat. Bevorzugt steht R¹ für Isobutyl.

Das in Schritt a) des erfindungsgemäßen Verfahrens erhaltene Reaktionsgemisch kann weitere Komponenten enthalten, wie nicht umgesetztes 3-Methylbut-3-en-1-ol (III), nicht umgesetzten Aldehyd (IV), Wasser, organisches Lösungsmittel, etc.

Vorzugsweise enthält das in Schritt a) erhaltene Reaktionsgemisch das 2-substituierte 4-Hydroxy-4-methyl-tetrahydropyran der Formel (I) in einer Menge von 50 bis 90 Gew.-%, besonders bevorzugt 60 bis zu etwa 80 Gew.-%, bezogen auf das Gesamtgewicht des Reaktionsgemischs.

Vorzugsweise enthält das in Schritt a) erhaltene Reaktionsgemisch die Verbindungen der Formeln (V.1), (V.2) und (V.3) in einer Gesamtmenge von 5 bis 20 Gew.-%, besonders bevorzugt 5 bis zu etwa 15 Gew.-%, bezogen auf das Gesamtgewicht des Reaktionsgemischs.

Vorzugsweise enthält das in Schritt a) erhaltene Reaktionsgemisch die DioxanVerbindung der Formel (VI) in einer Gesamtmenge von 5 bis 20 Gew.-%, besonders bevorzugt 5 bis zu etwa 15 Gew.-%, bezogen auf das Gesamtgewicht des Reaktionsgemischs.

In einer typischen Zusammensetzung enthält das in Schritt a) erhaltene Reaktionsgemisch die folgenden Verbindungen, jeweils bezogen auf das Gesamtgewicht des Reaktionsgemischs:
Isovaleraldehyd: 0 - 5 Gew.%,
Isoprenol: 0 - 10 Gew.%,
Dihydropyran-Isomere (V.a - c): 5 - 15 Gew.-%,
1,3-Dioxan (VI): 5 - 15 Gew.-%,
Acetal (VII): 0 - 5 Gew.-%,
trans-(I): 15 - 22 Gew.-%,
cis-(I): 45 - 65 Gew.-%,
Wasser: 2 - 10 Gew.-%.

Bevorzugt enthält das in Schritt a) erhaltene Reaktionsgemisch die 2-substituierten 4-Hydroxy-4-methyl-tetrahydropyrane der Formel (I) in Form von Gemischen der cis-Diastereomeren der Formel cis-(I) und der trans-Diastereomeren der Formel trans-(I) 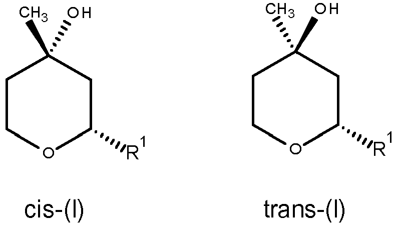 wobei das Diastereomerenverhältnis des cis-Diasteromeren cis-(I) zum trans-Diastereomeren trans-(I) bevorzugt 65 zu 35 bis 95 zu 5, besonders bevorzugt 70 zu 30 bis 85 zu 15 beträgt und R¹ die weiter oben angegebenen Bedeutungen hat.

Bevorzugt enthält das in Schritt a) erhaltene Reaktionsgemisch 2-Isobutyl-4-hydroxy-4-methyl-tetrahydropyran in Form von Gemischen des cis-Diastereomeren der Formel cis-(I.a) und des trans-Diastereomeren der Formel trans-(I.a) 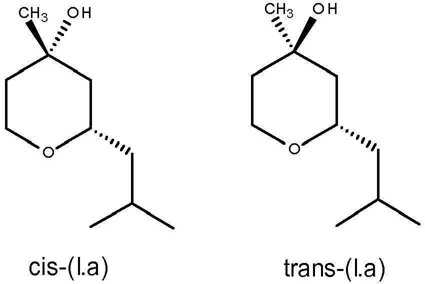 wobei das Diastereomerenverhältnis des cis-Diasteromeren cis-(I.a) zum trans-Diastereomeren trans-(I.a) bevorzugt 65 zu 35 bis 95 zu 5, besonders bevorzugt 70 zu 30 bis 85 zu 15 beträgt.

Derartige Gemische eignen sich aufgrund ihrer besonderen geruchlichen Eigenschaften in besonderem Masse zur Verwendung als Aromachemikalien, beispielsweise als Komponente mit Maiglöckchenduft zur Herstellung von Riechstoffkompositionen.

### Schritt b)

Das zur Auftrennung in Schritt b) eingesetzte Reaktionsprodukt aus Schritt a) enthält, bezogen auf das Gesamtgewicht, typischerweise 45 bis 65 Gew.-% der cis-Diastereomeren cis-(I), 15 bis 22 Gew.-% der trans-Diastereomeren trans-(I), 10 bis 30 Gew.-% niedriger als die Verbindungen (I) siedende Verbindungen, 1 bis 3 Gew.-% höher als die Verbindungen (I) siedende Verbindungen. Das Reaktionsprodukt aus Schritt a) ist vorzugsweise im Wesentlichen frei von Verbindungen, die einen Siedepunkt nahe dem der stereoisomeren Verbindungen (I) aufweisen. Im Wesentlichen frei von Verbindungen, die einen Siedepunkt nahe dem der stereoisomeren Verbindungen (I) aufweist, bedeutet im Rahmen der Erfindung, dass das Reaktionsprodukt aus Schritt a) höchstens 1 Gew.-%, besonders bevorzugt höchstens 0,5 Gew.-%, insbesondere höchstens 0,1 Gew.-% an Verbindungen enthält, die einen Siedepunkt nahe dem der stereoisomeren Verbindungen (I) aufweisen.

Bevorzugt enthält das zur Auftrennung in Schritt b) eingesetzte Reaktionsprodukt aus Schritt a) 45 bis 65 Gew.-% des cis-Diastereomeren von 2-Isobutyl-4-hydroxy-4-methyl-tetrahydropyran der Formel cis-(I.a), 15 bis 20 Gew.-% des trans-Diastereomeren der Formel trans-(I.a), 10 bis 25 Gew.-% niedriger als die Verbindungen (I) siedende Verbindungen, 1 bis 3 Gew.-% höher als die Verbindungen (I) siedende Verbindungen.

Bevorzugt wird in Schritt b) des erfindungsgemäßen Verfahrens das Reaktionsgemisch aus Schritt a) einer destillativen Auftrennung unterzogen. Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc. und Kombinationen davon.

Die Destillationskolonnen können trennwirksame Einbauten aufweisen, die vorzugsweise ausgewählt sind unter Trennböden, geordnete Packungen, z. B. Blech- oder Gewebepackungen, wie Sulzer Mellapak®, Sulzer BX, Montz B1 oder Montz A3 oder Kühni Rombopak, oder regellose Schüttungen von Füllkörpern, wie z. B. Dixon-Ringen, Raschig-Ringen, High-Flow-Ringen oder Raschig-Super-Ringen. Besonders bewährt haben sich geordnete Packungen, vorzugsweise Blech- oder Gewebepackungen, mit einer spezifischen Oberfläche von 100 bis 750 m²/m³, insbesondere 250 bis 500 m²/m³. Sie gestalten hohe Trennleistungen bei niedrigen Druckverlusten.

Vorzugsweise wird zur Auftrennung in Schritt b) eine Vorrichtung eingesetzt, die
- eine Zulaufsäule mit oberhalb der Zulaufstelle gelegenem Verstärkungsteil und unterhalb der Zulaufstelle gelegenem Abtriebsteil,
- eine mit dem oberen Ende des Verstärkungsteils kommunizierende obere Vereinigungssäule und eine mit dem unteren Ende des Abtriebsteils kommunizierende untere Vereinigungssäule, und
- eine mit der oberen Vereinigungssäule und der unteren Vereinigungssäule kommunizierende Abzugssäule umfasst.

Vorzugsweise erfolgt die Auftrennung in Schritt b), indem man
i) das Reaktionsprodukt aus Schritt a) in eine Zulaufsäule mit oberhalb der Zulaufstelle gelegenem Verstärkungsteil und unterhalb der Zulaufstelle gelegenem Abtriebsteil einführt,
ii) eine mit dem oberen Ende des Verstärkungsteils kommunizierende obere Vereinigungssäule mit Kondensator am oberen Säulenende und eine mit dem unteren Ende des Abtriebsteils kommunizierende untere Vereinigungssäule mit Aufheizer am unteren Säulenende vorsieht,
iii) eine mit der oberen Vereinigungssäule und der unteren Vereinigungssäule kommunizierende Abzugssäule vorsieht, die wenigstens einen Seitenabzug aufweist,
iv) aus der Abzugssäule am Kopf oder im oberen Bereich leichter als die 2-substituierten 4-Hydroxy-4-methyl-tetrahydropyrane (I) siedende Verbindungen abzieht, als wenigstens ein Seitenabzug zumindest einen Teil der 2-substituierten 4-Hydroxy-4-methyl-tetrahydropyrane (I) abzieht und im Sumpf oder im unteren Bereich der unteren Vereinigungssäule die 2-substituierten 4-Hydroxy-4-methyl-tetrahydropyrane (I) abzieht, die nicht als Seitenabzug abgezogen werden und die höher als die 2-substituierten 4-Hydroxy-4-methyl-tetrahydropyrane (I) siedenden Verbindungen abzieht.

In einer bevorzugten Ausführungsform enthält der aus der Abzugssäule am Kopf oder im oberen Bereich entnommene Abzug:
- zumindest einen Teil oder die Gesamtmenge der in dem Reaktionsprodukt aus Schritt a) enthaltenen Verbindungen (V.1), (V.2) und (V.3),
- zumindest einen Teil oder die Gesamtmenge der in dem Reaktionsprodukt aus Schritt a) enthaltenen Dioxanverbindung (VI),
- falls vorhanden, nicht umgesetztes 3-Methylbut-3-en-1-ol der Formel (III),
- falls vorhanden, nicht umgesetzten Aldehyd (IV),
- geringe Mengen oder keine 4-Hydroxy-4-methyl-tetrahydropyrane (I),
- Wasser.

In einer besonders bevorzugten Ausführungsform werden zur Umsetzung in Schritt a) 3-Methylbut-3-en-1-ol der Formel (III) und Isovaleraldehyd (IV) eingesetzt. Dann enthält der aus der Abzugssäule am Kopf oder im oberen Bereich entnommene Abzug:
- zumindest einen Teil oder die Gesamtmenge der in dem Reaktionsprodukt aus Schritt a) enthaltenen Verbindungen (V.1), (V.2) und (V.3), worin R¹ für Isobutyl steht,
- zumindest einen Teil oder die Gesamtmenge der in dem Reaktionsprodukt aus Schritt a) enthaltenen Dioxanverbindung (VI), worin R¹ für Isobutyl steht,
- falls vorhanden, nicht umgesetztes 3-Methylbut-3-en-1-ol der Formel (III),
- falls vorhanden, nicht umgesetzten Isovaleraldehyd (IV),
- geringe Mengen oder kein 2-(2-Methylpropyl)-4-hydroxy-4-methyl-tetrahydropyran der Formel (la),
- Wasser.

Das so erhaltene Kopfprodukt kann einer Phasentrennung zur Abtrennung der Hauptmenge des Wassers unterzogen werden. Abgesehen von einer solchen Phasentrennung kann das so erhaltene Kopfprodukt in der Regel ohne weitere Aufarbeitung zur Hydrierung in Schritt c) eingesetzt werden. Gewünschtenfalls kann das Kopfprodukt einer weiteren Aufarbeitung zur Abtrennung wenigstens eines Teils der von den Verbindungen (V.1), (V.2), (V.3) und (VI) verschiedenen Komponenten unterzogen werden. Dazu kann das Kopfprodukt z. B. einer weiteren destillativen Auftrennung unterzogen werden.

In einer bevorzugten Ausführungsform wird aus der Abzugssäule ein Seitenstrom oder werden aus der Abzugssäule zwei Seitenströme abgezogen. In einer speziellen Ausführung wird aus der Abzugssäule nur ein Seitenstrom abgezogen.

Werden in Schritt b) mehrere Abzüge entnommen, die 2-substituierte 4-Hydroxy-4-methyl-tetrahydropyrane (I) enthalten, z. B. zwei verschiedene Seitenabzüge oder ein Seitenabzug und ein Sumpfabzug, so unterscheiden sich diese in der Regel hinsichtlich der Zusammensetzung der Stereoisomeren. Somit gelingt die Isolierung einer gegenüber dem Reaktionsprodukt aus Schritt a) an cis-Diastereomeren angereicherten Fraktion und einer an trans-Diastereomeren angereicherten Fraktion. Bei ausreichender Trennleistung der eingesetzten Destillationsvorrichtung kann gewünschtenfalls zumindest eines der Diastereomeren in reiner Form erhalten werden.

Die Zulaufsäule, Abzugssäule, obere Vereinigungssäule und untere Vereinigungssäule können diskrete Bauelemente sein oder als Abschnitt oder Kammer einer Destillationskolonne ausgebildet sein, die mehrere Funktionen vereint. Der Ausdruck "kommunizierende Kolonnen" bedeutet, dass zwischen ihnen ein Austausch sowohl von aufsteigenden Brüden als auch von ablaufendem Kondensat erfolgt.

In einer bevorzugten Ausführungsform des erfindungsgemäßen Verfahrens erfolgt die destillative Auftrennung in Schritt b) in einer Anordnung von Destillationskolonnen, die eine Trennwandkolonne oder eine Zusammenschaltung von mindestens zwei thermisch gekoppelten konventionellen Destillationskolonnen umfasst.

Trennwandkolonnen sind spezielle Destillationskolonnen mit mindestens einer Zulaufstelle und mindestens drei Entnahmestellen, bei denen sich zwischen Verdampfer und Kondensator der sogenannte Rektifizierbereich befindet, in dem ein Teil des im Kondensator gebildeten Kondensats sich flüssig als Rücklauf im Gegenstrom zu den aus der Verdampfungseinrichtung aufsteigenden Dämpfen abwärts bewegt und welche in einem Teilbereich der Kolonne unterhalb und/oder oberhalb der Zulaufstelle mindestens eine in Längsrichtung wirkende Trenneinrichtung (Trennwand) zur Verhinderung einer Quervermischung von Flüssigkeits- und/oder Brüdenstrom (Dampfstrom) enthält und welche somit eine destillative Trennung von Stoffgemischen ermöglichen. Das Grundprinzip der Trennwandkolonnen ist seit langem bekannt und beispielsweise in der US 2,471,134, in der EP-A-0 122 367 oder in G. Kaibel, Chem. Eng. Technol. Vol. 10, 1987, Seite 92 bis 98 beschrieben.

Der allgemeine Grundaufbau einer Trennwandkolonne umfasst mindestens eine seitliche Zulaufstelle auf einer Seite der Trennwand und mindestens drei Entnahmestellen, von denen sich mindestens eine jenseits der Trennwand befindet. Da bei dieser Bauart im Bereich der Trennwand eine Quervermischung von Flüssigkeits- und/oder Brüdenstrom verhindert wird, ist es möglich, die Seitenprodukte in reiner Form zu erhalten. Hierdurch verringert sich bei der Auftrennung von Vielstoffgemischen im Allgemeinen die Zahl der insgesamt benötigten Destillationskolonnen. Zudem können beim Einsatz von Trennwandkolonnen gegenüber einer einfachen Hintereinanderschaltung zweier konventioneller Destillationskolonnen Investitionskosten sowie Energie eingespart werden (siehe M. Knott, Process Engineering, Vol. 2, 1993, February, Seite 33 bis 34).

Als konventionelle Destillationskolonnen werden im Sinne der Erfindung alle Destillationskolonnen bezeichnet, welche keine Trennwand enthalten. Bei thermisch gekoppelten konventionellen Destillationskolonnen werden Massen- und Energieströme wechselseitig ausgetauscht. Somit ist gegenüber einer einfachen Hintereinanderschaltung konventioneller Destillationskolonnen eine deutliche Einsparung von Energie möglich. Bevorzugt als Alternative zur Trennwandkolonne ist eine Verschaltung zweier thermisch gekoppelter Destillationskolonnen. Eine Übersicht verschiedener Anordnungen ist beispielsweise in G. Kaibel et al., Chem.-Ing.-Tech., Vol. 61, 1989, Seite 16 bis 25 und G. Kaibel et al., Gas Separation & Purification, Vol. 4, 1990, June, Seiten 109 bis 114, gegeben.

In einer ersten bevorzugten Ausführungsformen verwendet man zur Destillation eine Destillationskolonne mit einer thermisch gekoppelten Vorkolonne, d. h. die Abzugssäule, die obere Vereinigungssäule und die untere Vereinigungssäule sind als einstückige Destillationskolonne ausgebildet, und die Zulaufsäule ist als Vorkolonne zur Destillationskolonne ausgebildet. In einer zweiten bevorzugten Ausführungsform verwendet man eine Destillationskolonne mit einer thermisch gekoppelten Nachkolonne, d. h. die Zulaufsäule, die obere Vereinigungssäule und die untere Vereinigungssäule sind als einstückige Destillationskolonne ausgebildet und die Abzugssäule ist als Nachkolonne zu der Destillationskolonne ausgebildet. Destillationskolonnen mit beigeschalteten Hilfskolonnen sind bekannt und z. B. in Chem. Eng. Res. Des., Part A: Trans IChemE, März 1992, S. 118-132, "The design and optimisation of fully thermally coupled distillation columns", beschrieben.

Es hat sich als günstig erwiesen, aus dem Reaktionsprodukt aus Schritt a) vor dem Einführen in die Zulaufsäule zumindest einen Teil der leichter als die 2-substituierten 4-Hydroxy-4-methyl-tetrahydropyrane (I) siedenden Verbindungen zu entfernen. In einer speziellen Ausführungsform wird daher zur destillativen Auftrennung des Reaktionsprodukts aus Schritt a) eine Anordnung von Destillationskolonnen eingesetzt, die eine vorgeschaltete konventionelle Destillationskolonne und eine nachgeschaltete Trennwandkolonne oder eine nachgeschaltete Zusammenschaltung von zwei thermisch gekoppelten konventionellen Destillationskolonnen umfasst.

Bevorzugt wird zur destillativen Auftrennung in Schritt b)
b1) das Reaktionsgemisch aus Schritt a) zunächst einer Auftrennung in einer konventionellen Destillationskolonne unterzogen wird, wobei ein erstes Kopfprodukt erhalten wird, das an den Verbindungen (V.1), (V.2) und (V.3) und der Dioxanverbindung (VI) angereichert ist und im Wesentlichen keine Verbindungen der allgemeinen Formel (I) enthält, und ein erstes Sumpfprodukt erhalten wird, das an den Verbindungen (V.1), (V.2) und (V.3) und der Dioxanverbindung (VI) abgereichert ist und die Hauptmenge der Verbindungen der allgemeinen Formel (I) enthält,
b2) das erste Sumpfprodukt aus Schritt b1) einer Auftrennung in einer Trennwandkolonne oder in einer Zusammenschaltung von zwei thermisch gekoppelten konventionellen Destillationskolonnen unterzogen wird, wobei ein zweites Kopfprodukt erhalten wird, dass die nicht im ersten Kopfprodukt enthaltenen Verbindungen (V.1), (V.2), (V.3) und (VI) sowie gegebenenfalls geringe Mengen der Verbindungen der allgemeinen Formel (I) enthält, ein Seitenstrom erhalten wird, der im Wesentlichen aus Verbindung der allgemeinen Formel (I) besteht und ein zweites Sumpfprodukt erhalten wird, das die Verbindungen der allgemeinen Formel (I) enthält, die nicht im Kopfprodukt und nicht im Seitenstrom enthalten sind.

Bevorzugt steht auch in der zuvor genannten Ausführungsform in den Verbindungen der Formeln (I), (V.1), (V.2), (V.3) und (VI) R¹ für Isobutyl.

Der Ausdruck, wonach das erste Kopfprodukt im Wesentlichen keine Verbindungen der allgemeinen Formel (I) enthält, bedeutet, dass der Anteil Verbindungen der allgemeinen Formel (I) am ersten Kopfprodukt höchstens 5 Gew.-%, besonders bevorzugt höchstens 2 Gew.-%, insbesondere höchstens 1 Gew.-%, speziell höchstens 0,1 Gew.-%, bezogen auf das Gesamtgewicht des ersten Kopfprodukts, beträgt. In einer speziellen Ausführung enthält das erste Kopfprodukt keine Verbindungen der allgemeinen Formel (I).

Das zweite Kopfprodukt kann beispielsweise 0,1 bis 25 Gew.-%, besonders bevorzugt 0,2 bis 20 Gew.-%, insbesondere 0,3 bis 15 Gew.-%, speziell 0,5 bis 10 Gew.-%, Verbindungen der allgemeinen Formel (I), bezogen auf das Gesamtgewicht des zweiten Kopfprodukts, enthalten.

In einer speziellen Ausführung besteht der Seitenstrom nur aus Verbindungen der allgemeinen Formel (I).

In einer speziellen Ausführung besteht das zweite Sumpfprodukt nur aus Verbindungen der allgemeinen Formel (I). Alternativ kann das zweite Sumpfprodukt Verbindungen enthalten, die höher sieden als die Verbindungen der allgemeinen Formel (I).

Vorzugsweise wird nach dieser Ausführungsform das erste Kopfprodukt (insbesondere die an Wasser abgereicherte organische Phase des ersten Kopfprodukts) und/oder das zweite Kopfprodukt zur Hydrierung in Schritt c) eingesetzt. Dabei ist es unkritisch, wenn das zweite Kopfprodukt noch geringe Mengen der Verbindungen der allgemeinen Formel (I) enthält, da diese die Hydrierung in Schritt c) in der Regel unverändert durchlaufen und anschließend gewünschtenfalls abgetrennt und verwertet werden können.

In der Regel werden sich bei dieser Ausführungsform das Seitenprodukt und das zweite Sumpfprodukt bezüglich des Anteils der Stereoisomeren der Verbindungen der Formel (I) unterscheiden.

### Schritt c)

Die Hydrierung der Verbindungen (V.1), (V.2) und (V.3) zu den entsprechenden 2-substituierten 4-Methyl-tetrahydropyranen (II) kann auf an sich herkömmliche Weise mit Hydrierkatalysatoren des Standes der Technik ausgeführt werden.

Die Hydrierung kann katalytisch entweder in der Gas- oder Flüssigphase erfolgen. Vorzugsweise wird die Hydrierung in Schritt c) des erfindungsgemäßen Verfahrens in flüssiger Phase in Gegenwart eines heterogenen Hydrierkatalysators und eines wasserstoffhaltigen Gases durchgeführt.

Als Hydrierkatalysatoren kommen prinzipiell alle zur Hydrierung von ungesättigten organischen Verbindungen geeigneten homogenen und heterogenen Katalysatoren in Betracht. Dazu zählen z. B. Metalle, Metalloxide, Metallverbindungen oder Gemische davon. Geeignete Hydrierkatalysatoren enthalten vorzugsweise wenigstens ein Übergangsmetall, bevorzugt aus den Nebengruppen I. und VI. bis VIII. des Periodensystems der Elemente. Dazu zählen vorzugsweise Cu, Cr, Mo, Mn, Re, W, Fe, Rh, Co, Ni, Pd, Pt, Ru, Zn oder deren Mischungen.

Die Katalysatoren können allein aus den Aktivkomponenten bestehen, oder die Aktivkomponenten können auf Trägern aufgebracht sein. Als Trägermaterialien eignen sich z. B. Al₂O₃, SiO₂, ZrO₂, TiO₂, Aktivkohle, ZnO, BaO und MgO oder Mischungen daraus.

Zur Erhöhung der katalytischen Aktivität können Fe, Co und bevorzugt Ni, auch in Form der Raney-Katalysatoren oder als Metallschwamm mit einer sehr großen Oberfläche verwendet werden.

Bevorzugt wird für die Hydrierung im Schritt c) des erfindungsgemäßen Verfahrens Palladium auf Kohlenstoff oder Platin auf Kohlenstoff eingesetzt. Des Weiteren kann man Raney-Nickel oder Raney-Cobalt vorteilhaft verwenden.

Andere geeignete Katalysatoren enthalten z. B. 80 bis 100 Gew.-% Nickel und/oder Cobalt und bis zu 20 Gew.-% aktivierende Metalle wie Kupfer und/oder Chrom. Besonders vorteilhaft werden solche Katalysatoren als Trägerkatalysatoren verwendet. Der Gehalt an katalytisch aktiven Metallen solcher Trägerkatalysatoren beträgt in der Regel 5 bis 80 Gew.-%, bezogen auf die Summe von katalytisch aktiven Metallen und Träger.

Die Katalysatoren können zur Hydrierung in Schritt c) als Formkörper eingesetzt werden. Beispiele umfassen Katalysatorextrudate, wie Stränge Rippstränge und andere Extrudatformen, Schalenkatalysatoren, Tabletten, Ringe, Kugeln, Splitt, etc.

Bevorzugt wird die Hydrierung in Schritt c) bei einer Temperatur von 20 bis 200 °C, vorzugsweise 40 bis 150 °C, insbesondere 50 bis 120 °C durchgeführt.

Sofern man die Umsetzung in der Gasphase durchführt, haben sich Drücke von 1 bis 100 bar, vorzugsweise 1,1 bis 50 bar, bewährt.

Sofern man die Umsetzung in der Flüssigphase durchführt, liegt der Druck vorzugsweise in einem Bereich von 2 bis 500 bar, besonders bevorzugt von 3 bis 300 bar, insbesondere von 4 bis 250 bar, speziell von 5 bis 200 bar.

Die Hydrierung in Schritt c) kann in einem Reaktor oder mehreren hintereinander geschalteten Reaktoren durchgeführt werden. Die Hydrierung kann diskontinuierlich oder kontinuierlich erfolgen. Zur diskontinuierlichen Hydrierung kann z. B. ein Druckgefäß eingesetzt werden. Geeignete Druckgefäße sind z. B. Autoklaven, die mit einer Vorrichtung zum Beheizen und zum Rühren des Reaktorinhalts ausgestattet sind. Bevorzugt erfolgt die Hydrierung in der Flüssigphase über einem Festbett, vorzugsweise in Sumpf- oder Rieselfahrweise oder in Form einer Suspensionskatalyse.

Die Hydrierung kann ohne oder mit Zusatz eines Lösungsmittels erfolgen. Als Lösungsmittel kommen Alkohole, Ether, Kohlenwasserstoffe, wie beispielsweise Methanol, Ethanol, Isopropanol, Dioxan, Tetrahydrofuran, n-Pentan, Hexan, Cyclohexan, Toluol, etc. in Frage. Bevorzugt erfolgt die Hydrierung in Schritt c) ohne Zusatz eines Lösungsmittels.

Zur Hydrierung in Schritt c) kann man die Fraktion, die wenigstens eine der Verbindungen (V.1), (V.2) oder (V.3) und wenigstens eine Dioxanverbindung (VI) enthält, mit einem wasserstoffhaltigen Gas und einem Hydrierkatalysator in Kontakt bringen. Geeignete wasserstoffhaltige Gase sind ausgewählt unter Wasserstoff und Gemischen von Wasserstoff mit wenigstens einem inerten Gas. Geeignete inerte Gase sind z. B. Stickstoff oder Argon. Bevorzugt wird zur Hydrierung in Schritt c) Wasserstoff in unverdünnter Form, üblicherweise in einer Reinheit von etwa 99,9 Vol.-%, eingesetzt.

Durch die Hydrierung in Schritt c) werden die im Ausgangsgemisch enthaltenen Verbindungen (V.1), (V.2) und (V.3) in 2-substituierte 4-Methyl-tetrahydropyrane (II) überführt. Bevorzugt enthält das zur Hydrierung eingesetzte Ausgangsgemisch Verbindungen der Formel (V.1), (V.2) und (V.3), wobei der Rest R¹ für Isobutyl steht. Dann werden durch die Hydrierung in Schritt c) die im Ausgangsgemisch enthaltenen Verbindungen (V.1), (V.2) und (V.3) in 2-Isobutyl-4-methyl-tetrahydropyran (II) (Dihydrorosenoxid) überführt.

Bevorzugt wird in Schritt c) ein Hydrierprodukt erhalten, wobei das Diastereomerenverhältnis des cis-Diastereomeren des 2-substituierten 4-Methyl-tetrahydropyrans (II) zum trans-Diastereomeren in einem Bereich von 60 zu 40 bis 95 zu 5, bevorzugt von 65 zu 35 bis 90 zu 10, liegt. Besonders bevorzugt wird in Schritt c) ein Hydrierprodukt erhalten, wobei das Diastereomerenverhältnis des cis-Diastereomeren des 2-Isobutyl-4-methyl-tetrahydropyrans (II) zum trans-Diastereomeren in einem Bereich von 60 zu 40 bis 95 zu 5, bevorzugt von 65 zu 35 bis 90 zu 10, liegt.

Derartige Gemische eignen sich aufgrund ihrer besonderen Geruchseigenschaften in besonderem Maße zur Verwendung als Aromachemikalien, beispielsweise als Komponente mit rosenduftartigem Charakter zur Herstellung von Riechstoffkompositionen.

### Schritt d)

Aus dem in Schritt c) erhaltenen Hydrierprodukt kann prinzipiell nach üblichen, dem Fachmann bekannten Reinigungsverfahren eine an 2-substituierten 4-Methyl-tetrahydropyranen (II) angereicherte Fraktion und eine an der wenigstens einen Dioxanverbindung (VI) angereicherte Fraktion isoliert werden.

Bevorzugt wird das in Schritt c) erhaltene Hydrierprodukt einer destillativen Auftrennung unterzogen. Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc. und Kombinationen davon.

Bevorzugt wird das in Schritt c) erhaltene Hydrierprodukt in Schritt d) einer destillativen Auftrennung in wenigstens einer Destillationskolonnen unterzogen, die mit trennwirksamen Einbauten versehen ist.

Bevorzugt wird in Schritt d) aus dem in Schritt c) erhaltenen Hydrierprodukt eine an 2-substituierten 4-Methyl-tetrahydropyranen (II) angereicherte Fraktion isoliert, wobei das Diastereomerenverhältnis des cis-Diastereomeren zum trans-Diastereomeren in einem Bereich von 60 zu 40 bis 100 zu 0, bevorzugt von 65 zu 35 bis 90 zu 10, liegt.

Besonders bevorzugt wird in Schritt d) aus dem in Schritt c) erhaltenen Hydrierprodukt eine an 2-Isobutyl-4-methyl-tetrahydropyran (II) angereicherte Fraktion isoliert, wobei das Diastereomerenverhältnis des cis-Diastereomeren zum trans-Diastereomeren in einem Bereich von 60 zu 40 bis 100 zu 0, bevorzugt von 65 zu 35 bis 90 zu 10, liegt.

Bevorzugt wird in Schritt d) aus dem in Schritt c) erhaltenen Hydrierprodukt eine an 2-substituierten 4-Methyl-tetrahydropyranen (II) angereicherte Fraktion isoliert, die einen Gehalt an Dioxanverbindungen der allgemeinen Formel (VI) 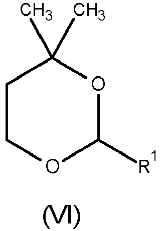 worin R¹ die zuvor angegebenen Bedeutungen hat und insbesondere für Isobutyl steht,
von höchstens 2 Gew.-%, besonders bevorzugt von höchstens 1 Gew.-%, ganz besonders bevorzugt von höchstens 0,1 Gew%, aufweist.

Die erfindungsgemäßen Zusammensetzungen und die nach dem erfindungsgemäßen Verfahren erhältlichen Zusammensetzungen eignen sich besonders vorteilhaft als Riechstoff oder zur Bereitstellung eines Riechstoffs.

Die erfindungsgemäßen Zusammensetzungen können für den Einsatz als Riechstoff mit wenigstens einem auf diesem Anwendungsgebiet üblichen Lösemittel beliebig verdünnt werden. Beispielhaft seien als geeignete Lösemittel genannt: Ethanol, Dipropylenglycol oder dessen Ether, Phthalate, Propylenglykole, oder Carbonate von Diolen, bevorzugt Ethanol. Auch Wasser ist als Lösemittel zur Verdünnung der erfindungsgemäßen Duftstoffkompositionen geeignet und kann vorteilhaft zusammen mit geeigneten Emulgatoren eingesetzt werden.

Die nach dem erfindungsgemäßen Verfahren erhaltenen Riechstoffe weisen auf Grund der strukturellen und chemischen Ähnlichkeit der Komponenten eine hohe Stabilität und Haltbarkeit auf. Die nach dem erfindungsgemäßen Verfahren erhältlichen Isomerengemische von 2-(2-Methylpropyl)-4-hydroxy-4-methyl-tetrahydropyran der Formel (la) zeichnen sich durch einen angenehmen Maiglöckchengeruch aus. Die nach dem erfindungsgemäßen Verfahren erhältlichen Isomerengemische von 2-(2-Methylpropyl)-4-methyl-tetrahydropyran der Formel (IIa) (Dihydrorosenoxid) zeichnen sich durch einen angenehmen rosenartigem Charakter aus.

Die nach dem erfindungsgemäßen Verfahren erhaltenen Riechstoffe eigenen sich zur Einarbeitung in kosmetische Zusammensetzungen sowie Gebrauchs- und Verbrauchsgüter bzw. Mittel wie sie im Folgenden näher beschrieben sind, wobei die Riechstoffe in die genannten Güter eingearbeitet oder auch auf solche aufgebracht sein können. Unter einer organoleptisch wirksamen Menge ist dabei wie im Rahmen der gesamten vorliegenden Erfindung insbesondere eine solche Menge zu verstehen, die bei sachgemäßer Anwendung ausreicht, beim Anwender bzw. Verbraucher einen Dufteindruck hervorzurufen.

Als kosmetische Zusammensetzungen sind alle üblichen kosmetischen Zusammensetzungen geeignet. Dabei handelt es sich bevorzugt um Parfum, Eau de Toilette, Deodorants, Seife, Duschgel, Badegel, Cremes, Lotions, Sonnenschutzmittel, Zusammensetzungen zur Reinigung und Pflege der Haare wie Haarshampoo, Spülung, Haargel, Haarfestiger in Form von Flüssigkeiten oder Schäumen und weitere Reinigungs- oder Pflegemittel für die Haare, Zusammensetzungen zur dekorativen Anwendung am menschlichen Körper, wie kosmetische Stifte, zum Beispiel Lippenstifte, Lippenpflegestifte, Abdeckstifte (Concealer), Wangenrouge (Blusher), Lidschattenstifte, Lippenkonturenstifte, Augenkonturenstifte, Augenbrauenstifte, Korrekturstifte, Sonnenschutzstifte, Anti-Akne-Stifte und vergleichbare Produkte sowie Nagellacke und weitere Produkte zur Nagelpflege.

Die nach dem erfindungsgemäßen Verfahren erhaltenen Riechstoffe eignen sich speziell für einen Einsatz in Parfums, z. B. als Eau de Toilette, Duschgele, Badegele und Körperdeodorants.

Sie eignen sich weiterhin zur Aromatisierung von Verbrauchs- oder Gebrauchsgütern, in die sie eingearbeitet bzw. auf die sie aufgebracht werden und ihnen dadurch einen angenehmen frischen grünen Akzent verleihen. Beispiele für Verbrauchs- oder Gebrauchsgüter sind: Raumluftdeodorants (Air Care), Reinigungsmitteln oder Pflegemitteln für Textilien (speziell Waschmittel, Weichspüler), Textilbehandlungsmittel wie beispielsweise Bügelhilfsmittel, Putzmittel, Reinigungsmittel, Pflegemittel zur Behandlung von Oberflächen, beispielsweise von Möbeln, Fußböden, Kücheneinrichtungen, Glasscheiben und Fenstern sowie Bildschirmen, Bleichen, Toilettensteine, Entkalkungsmittel, Düngemittel, Baustoffe, Schimmelentferner, Desinfektionsmittel, Produkte für die Auto- bzw. Fahrzeugpflege und dergleichen mehr.

Die folgenden Beispiele dienen der Verdeutlichung der Erfindung, ohne sie in irgendeiner Weise zu beschränken.

### BEISPIELE

Gaschromatographische Analysen wurden nach der folgenden Methode durchgeführt:
Säule: DB WAX 30 m x 0,32 mm;
FD 0,25 µm;
Injektortemperatur: 200 °C; Detektortemperatur 280 °C;
Temperaturprogramm:
   Anfangstemp.: 50 °C, mit 3 °C/min auf 170 °C,
   mit 20 °C/min auf 230 °C, 7 Min isotherm;
Retentionszeiten:
   Isovaleraldehyd t_{R} = 3,7 min
   cis-Dihydrorosenoxid t_{R} = 8,4 min
   trans-Dihydrorosenoxid t_{R} = 9,6 min
   4,4-Dimethyl-2-isobutyl-1,3-dioxan t_{R} = 11,9 min

Konzentrationen der erhaltenen Rohprodukte (Gew.-%) wurden GC-analytisch mit internem Standard ermittelt.

### Beispiel 1: (Hydrierung der isomeren Dihydropyrane V.1a - V.1 c)

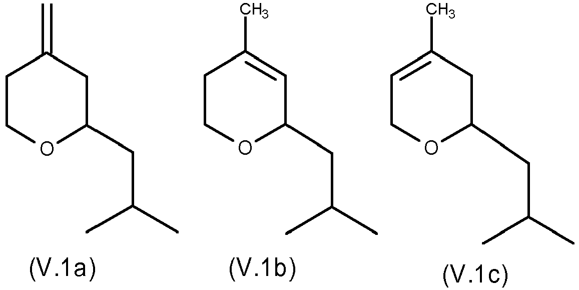

Eine Mischung der isomeren Dihydropyrane V.1a bis V.1 c (100 g, 0,65 mol) wurde bei Raumtemperatur in einem Autoklaven (max. Füllmenge 180 ml) vorgelegt und mit Palladium auf Kohle (5,8 % Pd, 50 % wasserfeucht) versetzt. Nach dem Schließen des Autoklaven wurde dieser drei Mal mit Stickstoff (20 bar) gespült, mit Wasserstoff ein Druck von 100 bar aufgepresst, der Rührer eingeschaltet (700 U/min) und der Autoklav auf 120 °C aufgeheizt. Bei 120 °C wurden 200 bar Wasserstoff aufgepresst und bei diesem Druck für weitere 15 h gerührt. Nach Abkühlen auf Raumtemperatur und Entspannen wurde der Austrag über einer Filternutsche (Por4 = Nennweite der Poren 10 - 16 µm) filtriert. Man erhielt das Rohprodukt (92 g) mit folgender Zusammensetzung: cis-Dihydrorosenoxid: 73,4 GC-FI% (t_{R} = 8,7 min); trans-Dihydrorosenoxid: 24,1 GC-FI% (t_{R} = 9,9 min). Nach anschließender destillativer Aufreinigung über einer 40 cm langen Füllkörperkolonne (Metallraschigringe) und einem Druck von 31 mbar wurde das Produkt bei einer Übergangstemperatur von 73 bis 74 °C mit einer Reinheit von 99,7 GC-FI% und einem Isomerenverhältnis von cis-Dihydrorosenoxid: trans-Dihydrorosenoxid = 3/1 erhalten.

### Beispiel 2:

Ein Gemisch aus Isovaleraldehyd (112,5 g, 1,31 mol), Isoprenol (125 g, 1,45 mol) und 12,5 g Wasser wurde in Gegenwart von 50 g des stark sauren Kationenaustauschers Amberlyst ® 131 umgesetzt, wie in Beispiel 1 der WO 2011/154330 beschrieben. Das erhaltene Reaktionsgemisch wurde einer destillativen Auftrennung in einer Anordnung aus einer konventionellen Destillationskolonne und einer Trennwandkolonne unterzogen.

Eine Mischung (gesamt = 150 g, repräsentativer Kopfabzug der Trennwandkolonne) aus Isovaleraldehyd (0,4 %), Isoprenol (0,8 %), den isomeren Dihydropyranen V.1a bis V.1 c (43,2 %), 4,4-Dimethyl-2-isobutyl-1,3-dioxan (42,0 %), Isoprenylether aus Pyranol (1,9 %) und den isomeren Pyranolen cis-(I.a) und trans-(I.a) (7,5 %) wurde in einem Autoklaven (max. Befüllung 180 ml) vorgelegt und mit Palladium auf Kohle (5,8 % Pd, 50 % wasserfeucht) versetzt. Nach dem Schließen des Autoklaven wurde dieser drei Mal mit Stickstoff (20 bar) gespült, mit Wasserstoff ein Druck von 150 bar aufgepresst, der Rührer eingeschaltet (700 U/min) und der Autoklav auf 120 °C aufgeheizt. Bei 120 °C wurden 200 bar Wasserstoff aufgepresst und bei diesem Druck für weitere 15 h gerührt. Nach Abkühlen auf Raumtemperatur und Entspannen auf 0 bar wurde der Austrag über einer Filternutsche (Por4 = Nennweite der Poren 10 - 16 µm) filtriert. Man erhielt das Rohprodukt (140 g) mit folgender Zusammensetzung: Isovaleraldehyd: 0,4 GC-FI% (t_{R} = 3,7 min); cis-Dihydrorosenoxid: 37,6 GC-FI% (t_{R} = 8,4 min); trans-Dihydrorosenoxid: 10,3 GC-FI% (t_{R} = 9,6 min); Dioxan: 36,9 GC-FI% (t_{R} = 11,9 min); IMTP: 3,1 GC-FI% (t_{R} = 27,0 min); Pyranol: 7,1 GC-FI% (t_{R} = 28,2 min). Nach anschließender destillativer Aufreinigung über eine 85 cm lange Füllkörperkolonne (Metallraschigringe) und einem Druck von 20 mbar wurde das Kopfprodukt bei einer Übergangstemperatur von 48 °C mit folgender Zusammensetzung erhalten: cis-Dihydrorosenoxid 1: 91,0 GC-FI% (t_{R} = 8,4 min); trans-Dihydrorosenoxid 2: 7,1 GC-FI% (t_{R} = 9,6 min); Dioxan: 0,6 GC-FI% (t_{R}= 11,9 min); 2-Methyl-2,4-butandiol 0,5 GC-FI% (t_{R} = 26,8 min).

### Beispiel 3:

Ein Gemisch aus Isovaleraldehyd (112,5 g, 1,31 mol), Isoprenol (125 g, 1,45 mol) und 12,5 g Wasser wurde in Gegenwart von 50 g des stark sauren Kationenaustauschers Amberlyst ® 131 umgesetzt, wie in Beispiel 1 der WO 2011/154330 beschrieben. Das erhaltene Reaktionsgemisch wurde einer destillativen Auftrennung in einer Anordnung aus einer konventionellen Destillationskolonne und einer Trennwandkolonne unterzogen.

Eine Mischung der Kopfabzüge der oben genannten Kolonnen (Gesamtmenge der Mischung: 100 g) aus Isovaleraldehyd (12,1 %), Isoprenol (10,7 %), den isomeren Dihydropyranen V.1a - V.1c (50,3 %), 4,4-Dimethyl-2-isobutyl-1,3-dioxan (20,8 %) und den isomeren Pyranolen cis-(I.a) und trans-(I.a) (6 %) wurde in einem Autoklaven (max. Befüllung 180 ml) vorgelegt und mit Raney-Nickel-Katalysator (wasserfeucht; 1 g) versetzt. Nach dem Schließen des Autoklaven wurde dieser drei Mal mit Stickstoff (20 bar) gespült, der Rührer eingeschaltet (700 U/min), mit Wasserstoff ein Druck von 5 bar aufgepresst, und der Autoklav auf 150 °C aufgeheizt. Bei 150 °C wurden 10 bar Wasserstoff aufgepresst und bei diesem Druck für weitere 20 h gerührt. Nach Abkühlen auf Raumtemperatur und Entspannen auf 0 bar wurde der Austrag über einer Filternutsche (Nennweite der Poren 10 - 16 µm) filtriert. Man erhielt das Rohprodukt mit folgender Zusammensetzung: Isovaleraldehyd: 0,6 GC-Gew.-% (t_{R} = 3,7 min); cis-Dihydrorosenoxid: 22,1 GC-Gew.-% (t_{R} = 8,0 min); Isoamylalkohol: 22,5 GC-FI% (t_{R} = 8,8 min); trans-Dihydrorosenoxid: 21,6 GC-Gew.-% (t_{R} = 9.2 min); Dihydropyranen V.1a - V.1c: 0,3 GC-Gew.-% (t_{R} = 9,4, 11,1, 11,8 min); Dioxan: 19,8 GC-Gew.-% (t_{R} = 11,5 min); Pyranol cis-(I.a) und trans-(I.a): 5,9 GC-Gew.-% (t_{R} = 27,3, 28,7 min).

Die destillative Aufarbeitung des Rohprodukts kann wie in Beispiel 1 oder 2 erfolgen.

### Beispiel 4:

Ein Gemisch aus Isovaleraldehyd (112,5 g, 1,31 mol), Isoprenol (125 g, 1,45 mol) und 12,5 g Wasser wurde in Gegenwart von 50 g des stark sauren Kationenaustauschers Amberlyst ® 131 umgesetzt, wie in Beispiel 1 der WO 2011/154330 beschrieben. Das erhaltene Reaktionsgemisch wurde einer destillativen Auftrennung in einer Anordnung aus einer konventionellen Destillationskolonne und einer Trennwandkolonne unterzogen.

Eine Mischung der Kopfabzüge der oben genannten Kolonnen (Gesamtmenge der Mischung: 50 g) aus Isovaleraldehyd (4,5 %), Isoprenol (11,3 %), den isomeren Dihydropyranen V.1a - V.1 c (35,3 %), 4,4-Dimethyl-2-isobutyl-1,3-dioxan (39,8 %) und den isomeren Pyranolen cis-(I.a) und trans-(I.a) (2,5 %) wurde in einem Autoklaven (max. Befüllung 180 ml) vorgelegt und mit Palladium auf Kohle (5 % Pd auf C, 50 % wasserfeucht; 2 g) sowie mit Methanol (100 ml) versetzt. Nach dem Schließen des Autoklaven wurde dieser drei Mal mit Stickstoff (20 bar) gespült, der Rührer eingeschaltet (700 U/min), mit Wasserstoff ein Druck von 5 bar aufgepresst, und der Autoklav auf 85 °C aufgeheizt. Bei 85 °C wurden 10 bar Wasserstoff aufgepresst und bei diesem Druck für weitere 15 h gerührt. Nach Abkühlen auf Raumtemperatur und Entspannen auf 0 bar wurde der Austrag über einer Filternutsche (Nennweite der Poren 10 - 16 µm) filtriert. Man erhielt das Rohprodukt mit folgender Zusammensetzung: Methanol: 42,0 GC-FI% (t_{R} = 3,4 min); 1,1-Dimethoxy-3-methyl-butan: 10,4 GC-FI% (4,6 min); Isovaleraldehyd: 0,5 GC-Gew.-% (t_{R} = 3,7 min); cis-Dihydrorosenoxid: 9,8 GC-Gew.-% (t_{R} = 8,0 min); Isoamylalkohol: 2,1 GC-FI% (t_{R} = 8.7 min); trans-Dihydrorosenoxid: 2,7 GC-Gew.-% (t_{R} = 9,1 min); Dioxan: 8,.0 GC-Gew.-% (t_{R} = 11,4 min); Pyranol cis-(I.a) und trans-(I.a): 1,0 GC-Gew.-% (t_{R} = 27,.3, 28,9 min).

Die destillative Aufarbeitung des Rohprodukts kann wie in Beispiel 1 oder 2 erfolgen.

## Patentansprüche

1. Verfahren zur Herstellung von 2-substituierten 4-Hydroxy-4-methyl-tetrahydropyranen der allgemeinen Formel (I) und von 2-substituierten 4-Methyl-tetrahydropyranen der allgemeinen Formel (II) 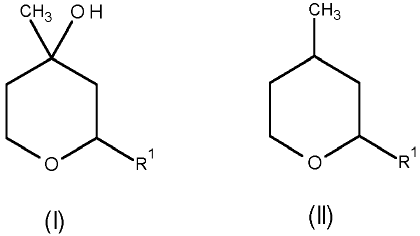 worin
R¹ für geradkettiges oder verzweigtes C₁-C₁₂-Alkyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) 3-Methylbut-3-en-1-ol der Formel (III) 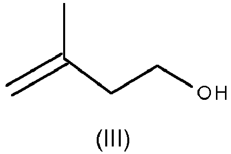 mit einem Aldehyd der Formel (IV)
R¹-CHO (IV)
wobei R¹ in der Formel (IV) die zuvor angegebene Bedeutung hat,
in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (I), wenigstens eine der Verbindungen (V.1), (V.2) oder (V.3) und wenigstens eine Dioxanverbindung (VI) enthält 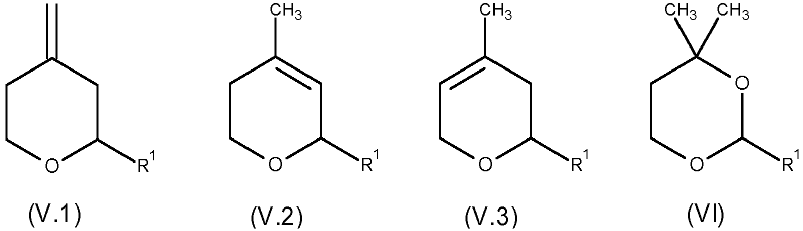 wobei R¹ in der Formel (VI) die zuvor angegebene Bedeutung hat,
b) das Reaktionsprodukt aus Schritt a) einer Auftrennung unter Erhalt einer an 2-substituierten 4-Hydroxy-4-methyl-tetrahydropyranen der allgemeinen Formel (I) angereicherten Fraktion und einer Fraktion, die wenigstens eine der Verbindungen (V.1), (V.2) oder (V.3) und wenigstens eine Dioxanverbindung (VI) enthält, unterzieht,
c) die Fraktion, die wenigstens eine der Verbindungen (V.1), (V.2) oder (V.3) und wenigstens eine Dioxanverbindung (VI) enthält, einer Hydrierung unterzieht,
d) aus dem in Schritt c) erhaltenen Hydrierprodukt eine an 2-substituierten 4-Methyl-tetrahydropyranen (II) angereicherte Fraktion und eine an der wenigstens einen Dioxanverbindung (VI) angereicherte Fraktion isoliert.

2. Verfahren nach einem der vorhergehenden Ansprüche, wobei der Rest R¹ für Isobutyl oder Phenyl steht.

3. Verfahren nach Anspruch 1 oder 2, bei dem die Umsetzung in Schritt a) in Gegenwart eines sauren Katalysators erfolgt, der ausgewählt ist unter Salzsäure, Schwefelsäure, Methansulfonsäure, p-Toluolsulfonsäure und stark sauren Kationenaustauschern.

4. Verfahren nach einem der vorhergehenden Ansprüche, wobei man in Schritt b) das Reaktionsprodukt aus Schritt a) einer destillativen Auftrennung unterzieht.

5. Verfahren nach Anspruch 4, wobei zur Auftrennung in Schritt b) eine Vorrichtung eingesetzt wird, die
- eine Zulaufsäule mit oberhalb der Zulaufstelle gelegenem Verstärkungsteil und unterhalb der Zulaufstelle gelegenem Abtriebsteil,
- eine mit dem oberen Ende des Verstärkungsteils kommunizierende obere Vereinigungssäule und eine mit dem unteren Ende des Abtriebsteils kommunizierende untere Vereinigungssäule, und
- eine mit der oberen Vereinigungssäule und der unteren Vereinigungssäule kommunizierende Abzugssäule
umfasst.

6. Verfahren nach einem der Ansprüche 4 oder 5, wobei die destillative Auftrennung in einer Anordnung von Destillationskolonnen erfolgt, die eine Trennwandkolonne oder eine Zusammenschaltung von mindestens zwei thermisch gekoppelten konventionellen Destillationskolonnen umfasst.

7. Verfahren nach einem der Ansprüche 4 bis 6, wobei zur destillativen Auftrennung des Reaktionsprodukts aus Schritt a) eine Anordnung von Destillationskolonnen eingesetzt wird, die eine vorgeschaltete konventionelle Destillationskolonne und eine nachgeschaltete Trennwandkolonne oder eine nachgeschaltete Zusammenschaltung von zwei thermisch gekoppelten konventionellen Destillationskolonnen umfasst.

8. Verfahren nach Anspruch 7, wobei zur destillativen Auftrennung in Schritt b)
b1) das Reaktionsgemisch aus Schritt a) zunächst einer Auftrennung in einer konventionellen Destillationskolonne unterzogen wird, wobei ein erstes Kopfprodukt erhalten wird, das an den Verbindungen (V.1), (V.2) und (V.3) und der Dioxanverbindung (VI) angereichert ist und im Wesentlichen keine Verbindungen der allgemeinen Formel (I) enthält, und ein erstes Sumpfprodukt erhalten wird, das an den Verbindungen (V.1), (V.2) und (V.3) und der Dioxanverbindung (VI) abgereichert ist und die Hauptmenge der Verbindungen der allgemeinen Formel (I) enthält,
b2) das erste Sumpfprodukt aus Schritt b1) einer Auftrennung in einer Trennwandkolonne oder in einer Zusammenschaltung von zwei thermisch gekoppelten konventionellen Destillationskolonnen unterzogen wird, wobei ein zweites Kopfprodukt erhalten wird, das die nicht im ersten Kopfprodukt enthaltenen Verbindungen (V.1), (V.2), (V.3) und (VI) sowie gegebenenfalls geringe Mengen der Verbindungen der allgemeinen Formel (I) enthält, ein Seitenstrom erhalten wird, der im Wesentlichen aus Verbindung der allgemeinen Formel (I) besteht und ein zweites Sumpfprodukt erhalten wird, das die Verbindungen der allgemeinen Formel (I) enthält, die nicht im Kopfprodukt und nicht im Seitenstrom enthalten sind.

9. Verfahren nach Anspruch 8, wobei das erste Kopfprodukt, vorzugsweise nach einer Phasentrennung zur Abtrennung der Hauptmenge des Wassers, und/oder das zweite Kopfprodukt zur Hydrierung in Schritt c) eingesetzt werden.

10. Verfahren nach einem der Ansprüche 8 oder 9, wobei das Seitenprodukt und das zweite Sumpfprodukt sich bezüglich des Anteils der Stereoisomeren der Verbindungen der Formel (I) unterscheiden.

11. Verfahren nach einem der vorhergehenden Ansprüche, wobei die Hydrierung in Schritt c) in Gegenwart eines Hydrierkatalysators erfolgt, der ausgewählt ist unter homogenen und heterogenen Katalysatoren, die wenigstens eine Metallkomponente, ausgewählt unter Metallen, Metalloxiden, Metallverbindungen oder Gemische davon enthalten, insbesondere Palladium auf Kohlenstoff, Platin auf Kohlenstoff, Raney-Nickel oder Raney-Cobalt.

12. Verfahren nach einem der vorhergehenden Ansprüche, wobei in Schritt d) das in Schritt c) erhaltene Hydrierprodukt einer destillativen Auftrennung unterzogen wird.

13. Verfahren nach einem der vorhergehenden Ansprüche, wobei in Schritt d) aus dem in Schritt c) erhaltenen Hydrierprodukt eine an 2-substituierten 4-Methyl-tetrahydropyranen (II) angereicherte Fraktion isoliert wird, wobei das Diastereomerenverhältnis des cis-Diastereomeren zum trans-Diastereomeren in einem Bereich von 60 zu 40 bis 100 zu 0, bevorzugt von 65 zu 35 bis 95 zu 10, liegt.

14. Verfahren nach einem der vorhergehenden Ansprüche, wobei in Schritt d) aus dem in Schritt c) erhaltenen Hydrierprodukt eine an 2-substituierten 4-Methyl-tetrahydropyranen (II) angereicherte Fraktion isoliert wird, die einen Gehalt an Dioxanverbindungen der allgemeinen Formel (VI) 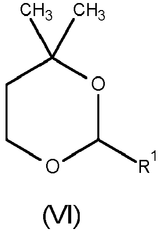
worin R¹ die zuvor angegebenen Bedeutungen hat und insbesondere für Isobutyl steht,
von höchstens 2 Gew.-%, besonders bevorzugt von höchstens 1 Gew.-%, aufweist.

15. Verfahren nach einem Anspruch 13 oder 14, wobei in Schritt d) eine an 2-Isopropyl-4-methyl-tetrahydropyran (II) angereicherte Fraktion isoliert wird.

## Claims

1. A process for preparing 2-substituted 4-hydroxy-4-methyltetrahydropyrans of the general formula (I) and of 2-substituted 4-methyltetrahydropyrans of the general formula (II) 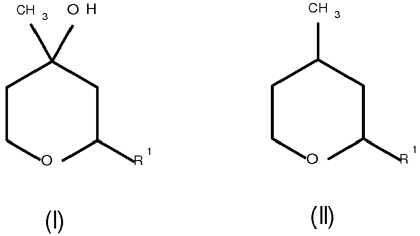 in which
R¹ is straight-chain or branched C₁-C₁₂-alkyl, unsubstituted or C1-C₁₂-alkyl- and/or C₁-C₁₂-alkoxy-substituted cycloalkyl having in total 3 to 20 carbon atoms or unsubstituted or C₁-C₁₂-alkyl- and/or C₁-C₁₂-alkoxy-substituted aryl having in total 6 to 20 carbon atoms,
in which
a) 3-methylbut-3-en-1-ol of the formula (III) 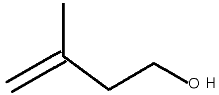 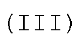 is reacted with an aldehyde of the formula (IV)
R¹-CHO (IV)
where R¹ in the formula (IV) has the meaning given above,
in the presence of an acidic catalyst, giving a reaction mixture which comprises at least one 2-substituted 4-hydroxy-4-methyltetrahydropyran of the general formula (I), at least one of the compounds (V.I), (V.2) or (V.3) and at least one dioxane compound (VI) 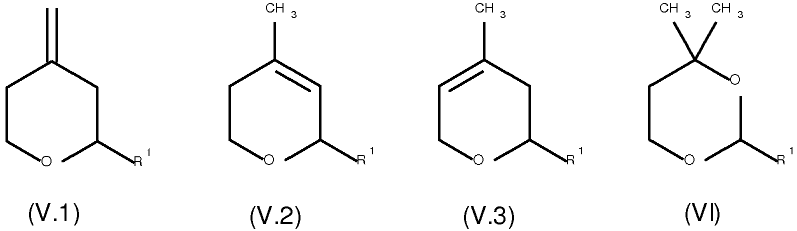 where R¹ in the formula (VI) has the meaning given above,
b) the reaction product from step a) is subjected to a separation, giving a fraction enriched in 2-substituted 4-hydroxy-4-methyltetrahydropyrans of the general formula (I) and a fraction which comprises at least one of the compounds (V.1), (V.2) or (V.3) and at least one dioxane compound (VI),
c) the fraction which comprises at least one of the compounds (V.1), (V.2) or (V.3) and at least one dioxane compound (VI) is subjected to a hydrogenation,
d) a fraction enriched in 2-substituted 4-methyltetrahydropyrans (II) and a fraction enriched in the at least one dioxane compound (VI) are isolated from the hydrogenation product obtained in step c).

2. The process according to any one of the preceding claims, where the radical R¹ is isobutyl or phenyl.

3. The process according to claim 1 or 2, in which the reaction in step a) takes place in the presence of an acidic catalyst which is selected from hydrochloric acid, sulfuric acid, methanesulfonic acid, p-toluenesulfonic acid and strongly acidic cation exchangers.

4. The process according to any one of the preceding claims, where, in step b), the reaction product from step a) is subjected to a distillative separation.

5. The process according to claim 4, where, for the separation in step b), a device is used which comprises
- a feed column with rectifying section positioned above the feed point, and stripping section positioned below the feed point,
- an upper combining column communicating with the upper end of the rectifying section, and a lower combining column communicating with the lower end of the stripping section, and
- a take-off column communicating with the upper combining column and the lower combining column.

6. The process according to either of claims 4 and 5, where the distillative separation takes place in an arrangement of distillation columns which comprises a dividing-wall column or an interconnection of at least two thermally coupled conventional distillation columns.

7. The process according to any one of claims 4 to 6, where, for the distillative separation of the reaction product from step a), an arrangement of distillation columns is used which comprises an upstream conventional distillation column and a downstream dividing-wall column or a downstream interconnection of two thermally coupled conventional distillation columns.

8. The process according to claim 7, where, for the distillative separation in step b),
b1) the reaction mixture from step a) is subjected firstly to a separation in a conventional distillation column, where a first top product is obtained which is enriched in the compounds (V.1), (V.2) and (V.3) and the dioxane compound (VI) and comprises essentially no compounds of the general formula (I), and a first bottom product is obtained which is depleted in the compounds (V.1), (V.2) and (V.3) and the dioxane compound (VI) and comprises the majority of the compounds of the general formula (I),
b2) the first bottom product from step b1) is subjected to a separation in a dividing-wall column or in an interconnection of two thermally coupled conventional distillation columns, where a second top product is obtained which comprises the compounds (V.1), (V.2), (V.3) and (VI) not present in the first top product, and also optionally small amounts of the compounds of the general formula (I), a side stream is obtained which consists essentially of compound of the general formula (I), and a second bottom product is obtained which comprises the compounds of the general formula (I) which are not present in the top product and are not present in the side stream.

9. The process according to claim 8, where the first top product, preferably after phase separation to remove the majority of the water, and/or the second top product are used for the hydrogenation in step c).

10. The process according to either of claims 8 and 9, where the side product and the second bottom product differ with regard to the fraction of stereoisomers of the compounds of the formula (I).

11. The process according to any one of the preceding claims, where the hydrogenation in step c) takes place in the presence of a hydrogenation catalyst which is selected from homogeneous and heterogeneous catalysts which comprise at least one metal component selected from metals, metal oxides, metal compounds or mixtures thereof, in particular palladium on carbon, platinum on carbon, Raney nickel or Raney cobalt.

12. The process according to any one of the preceding claims, where, in step d), the hydrogenation product obtained in step c) is subjected to a distillative separation.

13. The process according to any one of the preceding claims, where, in step d), a fraction enriched in 2-substituted 4-methyltetrahydropyrans (II) is isolated from the hydrogenation product obtained in step c), where the diastereomer ratio of the cis-diastereomer to the trans-diastereomer is in a range from 60:40 to 100:0, preferably from 65:35 to 95:10.

14. The process according to any one of the preceding claims, where, in step d), a fraction enriched in 2-substituted 4-methyltetrahydropyrans (II) is isolated from the hydrogenation product obtained in step c), said fraction having a content of dioxane compounds of the general formula (VI) 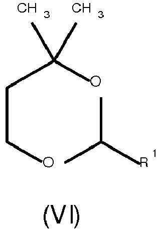 in which R¹ has the meanings given above and is in particular isobutyl,
of at most 2% by weight, particularly preferably of at most 1% by weight.

15. The process according to either of claims 13 and 14, where, in step d), a fraction enriched in 2-isopropyl-4-methyltetrahydropyran (II) is isolated.

## Revendications

1. Procédé de fabrication de 4-hydroxy-4-méthyl-tétrahydropyranes 2-substitués de formule générale (I) et de 4-méthyl-tétrahydropyranes 2-substitués de formule générale (II) 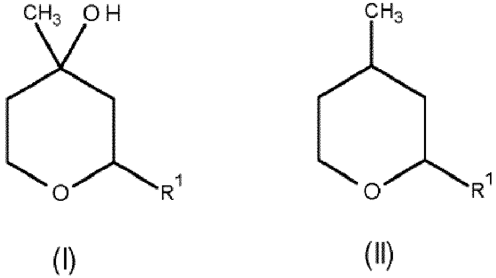 dans lesquelles
R¹ représente alkyle en C₁-C₁₂ linéaire ou ramifié, cycloalkyle contenant au total 3 à 20 atomes de carbone, non substitué ou substitué avec alkyle en C₁-C₁₂ et/ou alcoxy en C₁-C₁₂, ou aryle contenant au total 6 à 20 atomes de carbone, non substitué ou substitué avec alkyle en C₁-C₁₂ et/ou alcoxy en C₁-C₁₂,
selon lequel
a) du 3-méthylbut-3-én-1-ol de formule (III) 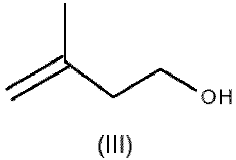 est mis en réaction avec un aldéhyde de formule (IV)
R¹-CHO (IV)
R¹ dans la formule (IV) ayant la signification indiquée précédemment,
en présence d'un catalyseur acide, un mélange réactionnel étant obtenu, qui contient au moins un 4-hydroxy-4-méthyl-tétrahydropyrane 2-substitué de formule générale (I), au moins un des composés (V.1), (V.2) ou (V.3), et au moins un composé de dioxane (VI) 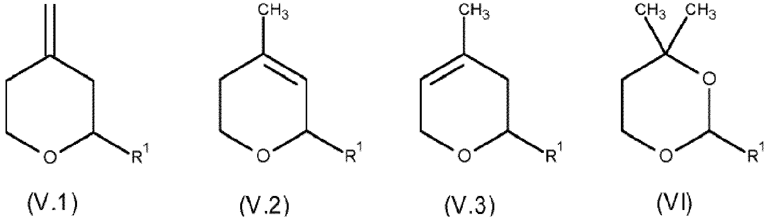 R¹ dans la formule (VI) ayant la signification indiquée précédemment,
b) le produit de réaction de l'étape a) est soumis à une séparation pour obtenir une fraction enrichie en 4-hydroxy-4-méthyl-tétrahydropyranes 2-substitués de formule générale (I) et une fraction qui contient au moins un des composés (V.1), (V.2) ou (V.3), et au moins un composé de dioxane (VI),
c) la fraction, qui contient au moins un des composés (V.1), (V.2) ou (V.3), et au moins un composé de dioxane (VI), est soumise à une hydrogénation,
d) à partir du produit de l'hydrogénation obtenu à l'étape c), une fraction enrichie en 4-méthyl-tétrahydropyranes 2-substitués (II) et une fraction enrichie en ledit au moins un composé de dioxane (VI) sont isolées.

2. Procédé selon l'une quelconque des revendications précédentes, dans lequel le radical R¹ représente isobutyle ou phényle.

3. Procédé selon la revendication 1 ou 2, dans lequel la réaction à l'étape a) a lieu en présence d'un catalyseur acide, qui est choisi parmi l'acide chlorhydrique, l'acide sulfurique, l'acide méthanesulfonique, l'acide p-toluènesulfonique et les échangeurs de cations acides forts.

4. Procédé selon l'une quelconque des revendications précédentes, dans lequel, à l'étape b), le produit de réaction de l'étape a) est soumis à une séparation par distillation.

5. Procédé selon la revendication 4, dans lequel, pour la séparation à l'étape b), un dispositif qui comprend
- une colonne d'alimentation comprenant une zone d'enrichissement située au-dessus de l'emplacement d'alimentation et une zone de rectification située en dessous de l'emplacement d'alimentation,
- une colonne de réunion supérieure communiquant avec l'extrémité supérieure de la zone d'enrichissement et une colonne de réunion inférieure communiquant avec l'extrémité inférieure de la zone de rectification, et
- une colonne de soutirage communiquant avec la colonne de réunion supérieure et la colonne de réunion inférieure,
est utilisé.

6. Procédé selon l'une quelconque des revendications 4 ou 5, dans lequel la séparation par distillation a lieu dans un agencement de colonnes de distillation qui comprend une colonne à paroi de séparation ou une interconnexion d'au moins deux colonnes de distillation classiques couplées thermiquement.

7. Procédé selon l'une quelconque des revendications 4 à 6, dans lequel, pour la séparation par distillation du produit de réaction de l'étape a), un agencement de colonnes de distillation est utilisé, qui comprend une colonne de distillation classique en amont et une colonne à paroi de séparation en aval ou une interconnexion de deux colonnes de distillation classiques couplées thermiquement en aval.

8. Procédé selon la revendication 7, dans lequel, pour la séparation par distillation à l'étape b),
b1) le mélange réactionnel de l'étape a) est tout d'abord soumis à une séparation dans une colonne de distillation classique, un premier produit de tête étant obtenu, qui est enrichi en les composés (V.1), (V.2) et (V.3) et en le composé de dioxane (VI), et ne contient essentiellement pas de composés de formule générale (I), et un premier produit de fond étant obtenu, qui est appauvri en les composés (V.1), (V.2) et (V.3) et en le composé de dioxane (VI), et contient la majorité des composés de formule générale (I),
b2) le premier produit de fond de l'étape b1) est soumis à une séparation dans une colonne à paroi de séparation ou une interconnexion de deux colonnes de distillation classiques couplées thermiquement, un second produit de tête étant obtenu, qui contient les composés (V.1), (V.2), (V.3) et (VI) non contenus dans le premier produit de tête, ainsi qu'éventuellement de petites quantités des composés de formule générale (I), un courant latéral étant obtenu, qui est essentiellement constitué de composé de formule générale (I), et un second produit de fond étant obtenu, qui contient les composés de formule générale (I) qui ne sont pas contenus dans le produit de tête et dans le courant latéral.

9. Procédé selon la revendication 8, dans lequel le premier produit de tête, de préférence après une séparation de phases pour la séparation de la majorité de l'eau, et/ou le second produit de tête sont utilisés pour l'hydrogénation à l'étape c).

10. Procédé selon l'une quelconque des revendications 8 ou 9, dans lequel le produit latéral et le second produit de fond diffèrent au niveau de la proportion des stéréoisomères des composés de formule (I).

11. Procédé selon l'une quelconque des revendications précédentes, dans lequel l'hydrogénation à l'étape c) a lieu en présence d'un catalyseur d'hydrogénation, qui est choisi parmi les catalyseurs homogènes et hétérogènes, qui contiennent au moins un composant métallique, choisi parmi les métaux, les oxydes de métaux, les composés de métaux ou leurs mélanges, notamment le palladium sur du carbone, le platine sur du carbone, le nickel de Raney ou le cobalt de Raney.

12. Procédé selon l'une quelconque des revendications précédentes, dans lequel, à l'étape d), le produit d'hydrogénation obtenu à l'étape c) est soumis à une séparation par distillation.

13. Procédé selon l'une quelconque des revendications précédentes, dans lequel, à l'étape d), à partir du produit d'hydrogénation obtenu à l'étape c), une fraction enrichie en 4-méthyl-tétrahydropyranes 2-substitués (II) est isolée, le rapport entre le diastéréomère cis et le diastéréomère trans se situant dans une plage allant de 60 sur 40 à 100 sur 0, de préférence de 65 sur 35 à 95 sur 10.

14. Procédé selon l'une quelconque des revendications précédentes, dans lequel, à l'étape d), à partir du produit d'hydrogénation obtenu à l'étape c), une fraction enrichie en 4-méthyl-tétrahydropyranes 2-substitués (II) est isolée, qui présente une teneur en composés de dioxane de formule générale (VI) 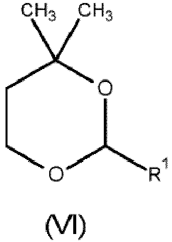 dans laquelle R¹ a les significations indiquées précédemment et représente notamment isobutyle,
d'au plus 2 % en poids, de manière particulièrement préférée d'au plus 1 % en poids.

15. Procédé selon l'une quelconque des revendications 13 ou 14, dans lequel, à l'étape d), une fraction enrichie en 2-isopropyl-4-méthyl-tétrahydropyrane (II) est isolée.
